# EUROPÄISCHE PATENTSCHRIFT

(11) **EP 0 400 499 B1**
(45) Veröffentlichungstag und Bekanntmachung des Hinweises auf die Patenterteilung: **06.04.1994**
(21) Anmeldenummer: 90109962.2
(22) Anmeldetag: 25.05.1990
(51) Int. Cl.: C07C 235/74, C07D 317/32, C07D 295/18, A61K 31/195, A61K 31/335, C07C 237/22

(54) **Neue Acylaminosäurederivate enthaltende Arzneimittel und Diätetika**
Medicine containing acylaminoacid derivatives and dietetic products
Médicament contenant des dérivés des acylamino acides et produits diététiques

(30) Priorität: 01.06.1989 DE 3917880
(43) Veröffentlichungstag der Anmeldung: 05.12.1990
(73) Patentinhaber: Kali-Chemie Aktiengesellschaft, D-30173 Hannover (DE); Kabi Pharmacia GmbH, D-91052 Erlangen (DE)
(72) Erfinder: Gerling, Klaus, D-3014 Laatzen 5 (DE); Heinemann, Henning, D-3160 Lehrte OT Aligse (DE); Meier, Andreas, D-3005 Hemmingen 1 (DE); Langer, Klaus, D-8520 Erlangen (DE)
(74) Vertreter: Lauer, Dieter, Dr.

(56) Entgegenhaltungen:
- WO-A-88/09789
- FR-A- 2 556 593

## Beschreibung

Die vorliegende Erfindung betrifft neue Acylaminosäurederivate mit wertvollen pharmakologischen Eigenschaften, insbesondere den Stickstoffstoffwechsel günstig beeinflussenden Eigenschaften, die Verwendung dieser Acylaminosäurederivate als Pharmazeutika und Diätetika insbesondere zur Behandlung und Prophylaxe von z.B. durch Leber-und/oder Nierenschädigungen bedingten Stickstoffstoffwechselstörungen in größeren Säugetieren, insbesondere Menschen, und Arzneimittel und Diätetika, welche als wirksamen Bestandteil Acylaminosäurederivate enthalten, sowie die Herstellung der Acylaminosäurederivate.

Es ist bereits bekannt, daß als Substitute für essentielle Aminosäuren die korrespondierenden α-Keto-Analoga dieser Aminosäuren dienen können. Die α-Keto-Analoga stehen mit wenigen Ausnahmen nach enzymatischer Transminierung als Eiweißbausteine im Organismus zur Verfügung (J.H. Close, N.Engl.J.Med. 290 (1974), S. 663-667).

Eine besondere Rolle im Rahmen der Proteinsynthese spielen Leucin und dessen α-Keto-Analogon 4-Methyl-2-oxovaleriansäure (α-Ketoleucin), da beide Substrate sich in ihrer Wirkungsweise synergistisch ergänzen. So wird einerseits die Proteinsynthese durch Leucin stimuliert und andererseits der Proteinabbau durch α-Ketoleucin inhibiert (M.E. Tischler, M. Desantels, A.L. Goldberg, J.Biol-Chem. 257 (1982), S. 1613-1621).

Weiterhin wird im Organismus beim Aufbau von α-Aminosäuren aus ihren entsprechenden α-Keto-Analoga Stickstoff verbraucht, wodurch zusätzlich die anfallende Menge harnpflichtiger Stickstoffverbindungen reduziert wird.

In Anwendung dieses Erkenntnisstandes wurden die Wirkungen von α-Keto- und α-Aminosäuren bei ganz unterschiedlichen Stoffwechselsituationen genutzt, insbesondere bei katabolen Zuständen, wie sie im Zusammenhang mit Leber- und Niereninsuffizienzen, Traumata, Sepsis und Fastenzuständen vorkommen.

Zusammensetzungen, welche α-Aminocarbonsäuren und α-Keto-Analoga von Aminocarbonsäuren enthalten, sind beispielsweise aus der FR-A-2 556 593 bekannt oder sind in Form oral zu verabreichender Zubereitungen bereits im Handel wie z.B. das Handelsprodukt ULTRAMIN^{R} (Hersteller Fa. Pfrimmer).

Zwar wurden durch die Anwendung dieser Produkte Therapieerfolge erzielt, doch zeigen sich sowohl bei ihrer Herstellung als auch bei ihrer oralen und parenteralen Anwendung noch verschiedene Probleme. So wird z.B. bei oraler Anwendung die verabreichte Menge dieser Aminosäuren und α-Keto-Analoga aufgrund des Resorptionsverhaltens leider nicht optimal ausgenutzt.

Bei parenteralem Einsatz, wie er z.B. für Leucin und Ketoleucin von G. Francois et.al. (Clin.Nutr.3 (1984), S.99-101) beschrieben wird, läßt sich der vorteilhafte Effekt einer Verminderung der Stickstoffausscheidung nur bei gleichzeitiger Gabe von Substraten des Kohlenhydratstoffwechsels erzielen. Bei der Herstellung entsprechender Infusionslösungen, die neben Aminosäuren und deren α-Keto-Analoga auch Substrate des Kohlenhydratstoffwechsels enthalten, besteht jedoch der Nachteil, daß sich beim Einsatz von reduzierenden Zuckern, wie z.B. Glucose, in solchen komplett zusammengestellten Infusionslösungen während der notwendigen Hitzesterilisation oder auch während der Lagerung Maillardprodukte bilden können.

Eine Aufgabe der Erfindung ist es, neue Aminosäurederivate mit wertvollen pharmakologischen und diätetischen Eigenschaften herzustellen, welche es gestatten, die vorgenannten Nachteile des Standes der Technik überwinden.

Eine weitere Aufgabe der Erfindung ist es, neue in der Aminosäurensubstitutionstherapie und zur Behandlung und Prophylaxe von Stickstoffstoffwechselstörungen verwendbare Pharmazeutika und/oder Diätetika zur Verfügung zu stellen, welche die vorgenannten Nachteile des Standes der Technik nicht aufweisen.

Die Erfindung betrifft neue Acylaminocarbonsäurederivate der allgemeinen Formel I 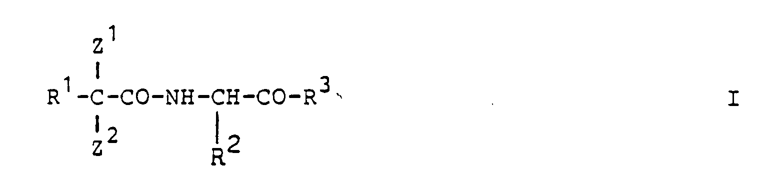
worin
- R¹: für einen organischen
Rest A 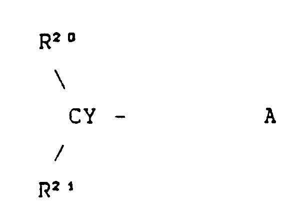 steht, worin
- Y: für Wasserstoff oder eine weitere Bindung steht, und falls
- R²⁰: Wasserstoff ist, R²¹ Isopropyl bedeutet, und falls
- R²⁰: Methyl ist, R²¹ Methyl oder Ethyl bedeutet,
- R²: den organischen Rest A′ 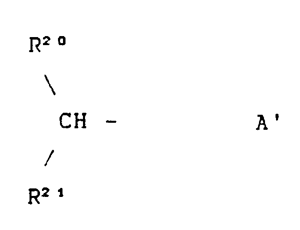 darstellt, worin R²⁰ und R²¹ obige Bedeutung besitzen,
- R³: für Hydroxy oder niederes Alkoxy oder für eine Aminogruppe B 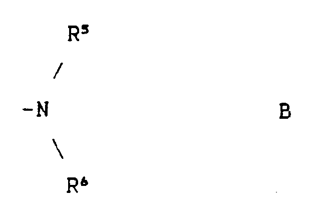 steht, worin
- R⁵: Wasserstoff oder niederes Alkyl bedeutet,
- R⁶: Wasserstoff, niederes Alkyl oder, falls R⁵ Wasserstoff ist, auch den Desaminorest einer biogenen Aminosäure bedeutet, oder worin
- R⁵ und R⁶: gemeinsam mit dem N-Atom, an welches sie gebunden sind, einen 3- bis 6-gliedrigen Heterocyclus bilden, und
- Z¹ und Z²: gemeinsam für Sauerstoff oder für eine physiologisch verträgliche Alkylendioxygruppe O-(CH₂)ₙ-O stehen, worin n 1-4 ist, oder
- Z¹ und Z²: je für eine physiologisch verträgliche R⁷-O-Gruppe stehen, worin R⁷ niederes Alkyl bedeutet, oder
- Z¹: für eine R⁷-O-Gruppe steht, worin R⁷ obige Bedeutung besitzt, und
- Z²: gemeinsam mit Y eine Bindung darstellt,
sowie Salze von solchen Verbindungen der Formel I, worin R³ für Hydroxy steht, mit einem physiologisch verträglichen Kation.

Sofern in den Verbindungen der Formel I die Substituenten niedere Alkylgruppen darstellen oder enthalten, können diese geradkettig oder verzweigt sein und vorzugsweise 1 bis 4, insbesondere 1 bis 2 Kohlenstoffatome enthalten.

Vorzugsweise stellen Z¹ und Z² in den Verbindungen der Formel I gemeinsam Sauerstoff dar, so daß sich Verbindungen der allgemeinen Formel Ia ergeben, 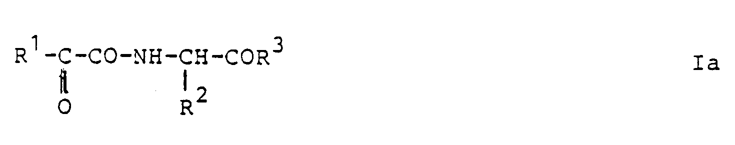
worin R¹ bis R³ die oben angegebene Bedeutung besitzen. Diese werden im folgenden als "Ketopeptide" bezeichnet.

Außer den Ketopeptiden der Formel Ia umfaßt die Erfindung auch deren physiologisch verträgliche Derivate, in denen die Ketogruppe durch Ketalbildung derivatisiert ist, das heißt, Verbindungen der Formel I, worin Z¹ und Z² je eine physiologisch verträgliche R⁷-O-Gruppe darstellen. In derartigen Gruppen ist die niedere Alkylgruppe R⁷ eine Alkylgruppe mit beispielsweise 1 bis 4, insbesondere 2 bis 3 Kohlenstoffatomen, welche bevorzugt geradkettig ist. Vorzugsweise stellt R⁷ Ethyl dar. Zur Ketalisierung kann auch ein Diol eingesetzt werden. In diesem Falle entstehen Verbindungen, worin Z¹ und Z² gemeinsam für eine Alkylendioxygruppe stehen. Bei den cyclischen Ketalen sind besonders jene bevorzugt, in denen die Alkylenkette 2 oder 3 Kohlenstoffatome enthält. Zu den Ketalderivaten von Verbindungen der Formel Ia gehören auch Verbindungen, in denen die Enolform dieser Verbindungen durch Ketalbildung derivatisiert ist, d.h. Verbindungen, in denen Z¹ eine R⁷-O-Gruppe darstellt und Z² zusammen mit Y eine Bindung bildet. Diese werden im folgenden als "Enoletherderivate" bezeichnet.

Ein in R¹ und/oder R² vorliegender Rest A′ stellt den Rest X einer biogenen α-Aminocarbonsäure X-CHNH₂-COOH aus der Gruppe Valin, Leucin und Isoleucin dar. Biogene Aminosäuren sind vorzugsweise jene L-α-Aminocarbonsäuren, die in biologischem Material vorgefunden werden. Solche Gruppen der Formel A′ sind dann der vom Valin stammende Rest (CH₃)₂CH-, der vom Leucin stammende Rest (CH₃)₂CH-CH₂- und der vom Isoleucin stammende Rest CH₃-CH₂-CH(CH₃)-.

Zur Vereinfachung der Bezeichnung der L-α-Aminocarbonsäuren die von der IUPAC-Nomenklaturkommission empfohlenen Dreibuchstabensymbole verwendet. Die in der vorliegenden Anmeldung verwendeten Symbole sind in der nachfolgenden Tabelle zusammengestellt.

| Aminosäure | Symbol |
|---|---|
| Valin | Val |
| Leucin | Leu |
| Isoleucin | Ile |
| Lysin | Lys |
| Arginin | Arg |
| Histidin | His |
| Ornithin | Orn |

Zweckmäßig ist R¹ ein organischer Rest, der auf die geschilderte Art ableitbar ist von L-Aminosäuren aus der Gruppe Val, Leu. Ebenso ist R² zweckmäßig ein entsprechender Rest, welcher ableitbar ist von L-Aminosäuren aus der Gruppe Val, Leu.

In den Verbindungen der Formel I steht das Kohlenstoffatom, an das R³ gebunden ist, auf der Oxidationsstufe eines Carbonsäure-C-Atoms. Das heißt, die Verbindungen stellen Säuren (R³ = OH) und deren Salze mit physiologisch verträglichen Kationen, bzw. deren Ester und Amide dar. Als besonders zweckmäßig erweisen sich Säuren der Formel I, worin R³ = OH, und deren Salze mit einem physiologisch verträglichen Kation. Als pharmazeutisch verwendbare Kationen eignen sich beispielsweise Metallkationen, wie Kationen von Alkalimetallen wie Natrium oder Kalium, Erdalkalimetallen wie Calcium oder Magnesium oder auch Zink.

Zweckmäßig ist als physiologisch verträgliches Kation auch eine Ammoniumgruppe C 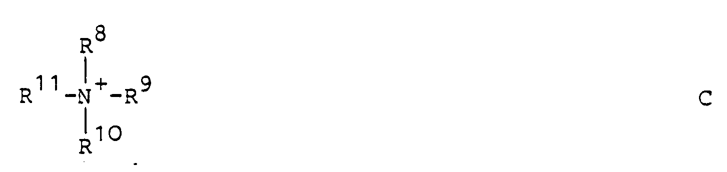
worin die Substituenten R⁸ bis R¹¹ unabhängig voneinander Wasserstoff oder niederes Alkyl bedeuten, oder zwei der Substituenten R⁸ bis R¹¹ gemeinsam eine C₄ - oder C₅-Alkylenkette und die anderen Substituenten Wasserstoff bedeuten, oder einer der Reste R⁸ bis R¹¹ den Desaminorest einer basischen biogenen α-Aminocarbonsäure darstellt und die anderen Reste Wasserstoff bedeuten. Niedere Alkylreste R⁸ bis R¹¹ können geradkettig oder verzweigt sein, wobei jedoch nur einer der Reste tert. Butyl bzw. nur zwei dieser Reste i-Alkylreste sein können. Als Desaminorest einer basischen biogenen α-Aminocarbonsäure wird im Rahmen der vorliegenden Erfindung ein solcher Rest verstanden, der sich aus einer basischen Aminosäure abzüglich der basischen Aminogruppe ergibt. Bevorzugte Ammoniumreste der Formel C sind NH₄⁺ sowie Ammoniumionen, welche die Desaminoreste von Lys, Arg, His oder Orn enthalten.

Sofern R³ eine niedere Alkoxygruppe bedeutet, kann diese 1 - 4 Kohlenstoffatome enthalten und geradkettig oder verzweigt sein.

Falls R³ eine Aminogruppe B bedeutet, können die Reste R⁵ und R⁶ unabhängig voneinander Wasserstoff oder niederes Alkyl sein. Niedere Alkylgruppen R⁵ oder R⁶ können geradkettig oder verzweigt sein und vorzugsweise 1 - 4, insbesondere 1 - 2 Kohlenstoffatome enthalten. R⁵ und R⁶ können auch gemeinsam mit dem Stickstoffatom, an welches sie gebunden sind, eine 3- bis 6-gliedrigen Heterocyclus bilden. Beispiele geeigneter Heterocyclen sind Aziridin, Pyrrolidin, Piperidin. Ferner kann, falls der Rest R⁵ Wasserstoff ist, R⁶ auch der Desaminorest einer biogenen Aminosäure sein. Derartige Verbindungen der Formel I stellen Tripeptidderivate dar.

Die Bezeichnung der Ketopeptide der allgemeinen Formel Ia bzw. deren Derivate wird in der vorliegenden Anmeldung in Übereinstimmung mit den IUPAC-Nomenklaturregeln unter Verwendung der weiter oben bereits definierten Dreibuchstaben Symbole in der Weise vorgenommen, daß man für die Bezeichnung einer α-Ketocarbonsäure, die sich von einer biogenen α-Aminocarbonsäure durch Ersatz der α-Aminogruppe und des an das α-C-Atom gebundenen Wasserstoffatoms durch eine Oxogruppe ableitet, das Dreibuchstaben-Symbol der zugrundeliegenden α-Aminocarbonsäure mit dem Präfix "Keto" versieht. Beispielsweise heißt das Keto-Analogon vom Leucin "Ketoleucin" (= 4-Methyl-2-Oxovaleriansäure). Eine Ketodipeptidsäure der Formel Ia, welche sich aus Ketoleucin (R¹ = Isopropylmethyl) und der Aminosäure Valin (R² = Isopropyl) ableitet, wird z.B. "Ketoleucylvalin" oder bei gleichzeitiger Angabe der Konfiguration der Aminosäure Valin "Ketoleucyl-L-Valin" genannt; die Kurzbezeichnung ist "CO-Leu-Val" bzw. "Ketoleu-Val". Der entsprechende Methylester von Ketoleucylvalin ist dementsprechend z.B. "Ketoleucylvalin-methylester" und lautet in Kurzschreibweise z.B. "CO-Leu-Val-OMe" bzw. "Ketoleu-Val-OMe".

Erfindungsgemäß bevorzugte Verbindungen sind:
Ketoleucylleucin oder Ketovalylvalin, sowie deren Salze mit physiologisch verträglichen Kationen der oben angegebenen Art.

Die Erfindung betrifft auch die Verwendung der erfindungsgemäßen Verbindungen der Formel I als Pharmazeutika und Diätetika in größeren Säugetieren, insbesondere Menschen. Die erfindungsgemäßen Verbindungen besitzen wertvolle pharmakologische Eigenschaften, sie eignen sich als Substitute für essentielle Aminosäuren und zeichnen sich insbesondere durch eine günstige Wirkung auf Stickstoffstoffwechselstörungen sowie eine gute Resorbierbarkeit und eine gute Stabilität in galenischen Zubereitungen aus.

Aufgrund ihrer physiologischen und pharmakologischen Eigenschaften sind die Aminosäurederivate der Formel I und ihre Salze mit physiologisch verträglichen Kationen zur Verwendung als Arzneimittel und Diätetika in der Aminosäuresubstitutionstherapie geeignet. Insbesondere können die Verbindungen eingesetzt werden bei der Behandlung und Prophylaxe von Störungen im N-Haushalt, z.B. von durch Leber- und/oder Niereninsuffizienz bedingten Stoffwechselstörungen und katabolen Störungen, welche im Zusammenhang mit Traumata, Sepsis und Fastenzuständen auftreten können.

Aufgrund ihrer chemischen Stabilität, auch in Gegenwart von Kohlehydraten, können Sie in beliebiger Weise in diätetische und/oder pharmazeutische Zubereitungen eingearbeitet werden und sind hervorragend zur Herstellung von Infusionslösungen, auch von reduzierende Kohlehydrate enthaltenden Infusionslösungen, geeignet.

Die Erfindung betrifft ferner Pharmazeutika und Diätetika insbesondere zur Anwendung als Aminosäurensubstitute und/oder zur Behandlung und Prophylaxe von Stickstoffstoffwechselstörungen, welche als wirksamen Bestandteil Verbindungen der Formel I oder deren Salze mit einem physiologisch verträglichen Kation neben üblichen physiologisch verträglichen Hilfs- und/oder Trägerstoffen enthalten. Erfindungsgemäß können die Verbindungen der Formel I und ihre physiologisch verträglichen Salze zusammen mit üblichen pharmazeutischen Hilfs- und/oder Trägerstoffen in festen oder flüssigen pharmazeutischen Zubereitungen enthalten sein. Als pharmazeutische Zubereitungen im Sinne der vorliegenden Erfindung werden sowohl Arzneimittel wie Diätetika verstanden. Als Beispiel fester Zubereitungen seien oral applizierbare Präparate wie Kapseln, Tabletten, Granulate oder Dragees genannt. Feste Präparate können pharmazeutisch übliche anorganische und/oder organische Trägerstoffe wie z.B. Talkum, Stärke oder Milchzucker neben pharmazeutisch üblichen Hilfsmitteln, beispielsweise Gleitmitteln wie Magnesiumstearat oder Tablettensprengmitteln enthalten. Flüssige Präparate wie Lösungen, Suspensionen oder Emulsionen können die üblichen Verdünnungsmittel wie Wasser und/oder Öle, z.B. Triglyceridgemische von gesättigten Pflanzenfettsäuren und/oder Suspensionsmittel wie z.B. Polyäthylenglycole und dergleichen oder auch weitere gelöste Nährstoffe, beispielsweise Kohlehydrate wie Glucose enthalten. Gewünschtenfalls können weitere Hilfsstoffe zugegeben werden wie z.B. Konservierungsmittel, Stabilisierungsmittel, Geschmackskorrigenzien und dergleichen.

Die Wirkstoffe können mit den pharmazeutischen Hilfs-und/oder Trägerstoffen in an sich bekannter Weise gemischt und formuliert werden. Zur Herstellung fester Arzneiformen können die Wirkstoffe mit den Trägerstoffen in üblicher Weise gemischt und naß oder trocken granuliert werden. Ein Granulat oder Pulver kann direkt in Kapseln oder Portionsbeutel abgefüllt oder in üblicher Weise zu Tablettenkernen verpreßt werden. Diese können gewünschtenfalls in bekannter Weise dragiert werden. Zur Herstellung von flüssigen Zubereitungen können die Verbindungen in an sich bekannter Weise in dem flüssigen Trägerstoff gelöst oder suspendiert werden. Für die parenterale Applikation bestimmte Lösungen oder Suspensionen können in an sich bekannter Weise sterilisiert werden.

Eine besondere Ausgestaltung von flüssigen Zubereitungen, welche als wirksamen Bestandteil Verbindungen der Formel I oder deren Salze mit einem physiolgisch verträglichen Kation enthalten, stellen insbesondere die für die parenterale Applikation bestimmten Lösungen dar. Diese parenteral applizierbaren Lösungen, d.h. Infusionslösungen, können in an sich bekannter Weise hergestellt werden. Hierzu löst man die gewünschte Menge einer oder mehrerer der erfindungsgemäßen Verbindungen der Formel I oder deren physiologisch verträgliche Salze unter Rühren in destilliertem Wasser (für Injektionszwecke), wobei durch gleichzeitige Begasung mit einem geeigneten Schutzgas, z.B. durch Stickstoffbegasung, ein möglichst weitgehender Ausschluß von Luftsauerstoff sichergestellt wird. Bei der Herstellung dieser wäßrigen Infusionslösungen wird auf ausreichende Isotonisierung der Lösungen geachtet. In Ausnahmefällen (z.B. bei unzureichender Löslichkeit) können weiterhin gegebenenfalls mit Wasser mischbare, als Cosolventien an sich bekannte Lösungsmittel und/oder weitere bekannte Hilfsstoffe für Infusionslösungen in begrenzter Menge zugesetzt werden. Die derart erhaltenen Lösungen werden anschließend zur Abscheidung von Partikeln und zur Keimzahlreduzierung über eine geeignete Filterkaskade mit einem Endfilter von z.B. etwa 0,2 µm Porendurchmesser gepumpt. Anschließend werden die hergestellten Infusionslösungen in bekannter Weise konfektioniert, d.h. man füllt beispielsweise in gespülte Glasflaschen ab, evakuiert danach den Kopfraum dieser befüllten Glasflaschen, verschließt letztere mit Gummistopfen und verbördelt abschließend. Die befüllten und verschlossenen Flaschen werden nachfolgend noch in einem Autoklaven unter Bedingungen hitzebehandelt, die die Sterilität des Produktes sichern.

Die Verbindungen der Formel I und ihre physiologisch verträglichen Salze bzw. diese enthaltende pharmazeutische Zubereitungen werden hervorragend resorbiert und gehen auch nach oraler Gabe zumindest teilweise intakt in das Blutplasma über. Durch ihre Verabreichung werden nicht nur günstige Blutplasmakonzentrationen an intakten Ketopeptiden auf der Formel Ia aufgebaut, sondern es werden in überraschender und vorteilhafter weise auch die nativen Blutplasmaspiegel der korrespondierenden α-Aminosäuren und der α-Keto-Analoga deutlich und lang anhaltend angehoben.

Neben pharmakologischen Vorteilen weisen die Verbindungen der Formel I und ihre physiologisch verträglichen Salze auch technologische Vorteile gegenüber entsprechenden Mischungen aus α-Aminosäuren und α-Ketosäuren auf. Säuren der Formel I sind, z.B. in Form ihrer Ornithin- oder Lysinsalze, gut wasserlöslich und erweisen sich auch unter drastischen Belastungen als extrem hitzestabil. Sie lassen sich daher problemlos den Bedingungen der für die Herstellung parenteraler Infusionslösungen notwendigen Hitzesterilisation unterwerfen, ohne daß dabei Zersetzungen auftreten. Eine hohe Langzeitstabilität gewährleistet eine gute Lagerfähigkeit der erfindungsgemäßen pharmazeutischen Zubereitungen.

Gegenüber Mischungen aus α-Aminosäuren und α-Ketosäuren bieten die Verbindungen der Formel I darüber hinaus besondere Vorteile bei der Herstellung von kompletten Infusionslösungen, welche zusätzlich Substrate des Kohlehydratstoffwechsels enthalten. Während z.B. bei der Zubereitung einer Glucose enthaltenden hitzesterilisierten Lösung aus α-Aminosäuren und α-Ketosäuren die Brauchbarkeit der sterilisierten Lösung durch Maillardreaktionen beeinträchtigt wird, treten diese Maillardreaktionen bei der gemeinsamen Verarbeitung von Verbindungen der Formel I mit Glucose zu hitzesterilisierten Lösungen nicht auf.

Die vorteilhaften Eigenschaften von Verbindungen der Formel I enthaltenden pharmazeutischen Zubereitungen werden in den nachfolgenden Versuchen demonstriert.

### Versuch 1

Die Resorptionskinetik der Verbindungen der Formel I soll am Beispiel des Ketoleucylleucins bei Ratten gezeigt werden. 12 Ratten (Spraque dawley, 400 g) erhielten eine orale Zufuhr von jeweils 1 g Ketoleucylleucin in Form des Bisornithates in 5 gew.-%-iger Glucoselösung. Nach bestimmten Zeitabständen wurden Blutproben entnommen und auf α-Ketoleucylleucin, auf α-Ketosäuren und α-Aminosäuren geprüft.

| | Konzentration in µmol/l nach | | | | | |
|---|---|---|---|---|---|---|
| | 0 min | 15 min | 30 min | 60 min | 120 min | 180 min |
| CO-Leu-Leu | 0 | 20 | 26 | 15 | 9 | 6 |
| CO-Leu | 20 | 91 | 135 | 91 | 77 | 58 |
| Leu | 185 | 715 | 795 | 715 | 715 | 610 |

Die in der Tabelle wiedergegebenen Versuchsergebnisse zeigen am Beispiel der oralen Gabe von Ketoleucylleucin, daß die Verbindungen der Formel I - zumindest teilweise - intakt resorbiert werden und eine drastische Erhöhung der Plasmakonzentration der entsprechenden α-Aminosäure, hier z.B. Leucin, und der entsprechenden α-Ketosäure, hier z.B. α-Ketoleucin, über längere Zeit erfolgt.

### Versuch 2

Zum Nachweis der überraschend hohen Resistenz von Ketopeptiden der Formel Ia gegen Maillard-Reaktion wurden aus der Gruppe der Ketopeptide das Magnesiumsalz des Ketoleucylvalins (CO-Leu-Val) und das Calciumsalz das Ketoisoleucylleucins (CO-Ile-Leu) jeweils im Vergleich zu der Mischung der korrespondierenden Aminosäuren unter nachfolgenden Bedingungen getestet.

In 20 %-iger Glucoselösung wird das Ketopeptid - oder zum Vergleich äquimolar das zugrundeliegende Aminosäure-Muster - in einer Konzentration von 1 Gew.-% über 60 Minuten konstant bei 121 °C gehalten. Die durch Maillard-Reaktionen hervorgerufene Bräunung wird durch Abnahme der Transmission bei 420 nm gemessen.

| | Transmission in % bei 420 nm nach | | | |
|---|---|---|---|---|
| | 0 min | 10 min | 30 min | 60 min |
| CO-Leu-Val (Mg-Salz) | 97,0 | 90,5 | 77,9 | 55,6 |
| Gemisch Leu + Val | 100,2 | 65,6 | 12,4 | 0 |
| CO-Leu-Ile (Ca-Salz) | 99,5 | 95,6 | 84,2 | 62,8 |
| Gemisch Leu + Ile | 97,8 | 66,4 | 5 | 0 |

Die in der voranstehenden Tabelle angegebenen Ergebnisse dokumentieren die hohe Überlegenheit von Ketopeptiden gegenüber den bloßen Mischungen der Aminosäuren.

### Versuch 3

Zur Stabilitätsprüfung wurden ausgewählte Ketopeptide der Formel Ia in wäßrigem Millieu bei pH 7 unter Stickstoffschutzgas über Zeiten von bis zu 60 Minuten auf 121 °C erhitzt. Nach den in der Tabelle angegebenen Zeitabschnitten für die Hitzebehandlung ermittelte man den verbliebenen, unzersetzten Anteil des Ketopeptides in % (bezogen auf die Menge des eingesetzten Ketopeptides). Die Analytik erfolgte durch hochauflösende Flüssigchromatographie (HPLC) mit:
- Säule:: Nucleosil^{R} 5 C18*, Länge = 20 cm, Innendurchmesser 4,6 mm;
- Eluens:: 0,05 m NaH₂PO₄ mit 50 bis 60 % Methanol;
- Detektion:: UV-Spektralfotometer bei 225 nm, Range 0,08.

(* Kieselgel, hydrophobiert durch C₁₈-Alkylreste, für die Reversed Phase Chromatographie)

| | prozentualer Restgehalt nach | | |
|---|---|---|---|
| | 10 min. | 30 min. | 60 min. |
| CO-Leu-Leu (Mg-Salz) Reinheit: 97,6 % | 97,0 % | 100 % | 99,2 % |
| CO-Leu-Val (Mg-Salz) Reinheit: 99,3 % | 101,5 % | 103,6 % | 102,0 % |
| CO-Ile-Leu (Ca-Salz) Reinheit: 88,5 % | 100 % | 100 % | 99,5 % |

Aus dem Test ist ersichtlich, daß die Ketodipeptide unter Hitzebelastung sehr stabil sind. Die Schwankungen um den 100 %-Wert liegen im Rahmen der Meßgenauigkeit.

Die Verbindungen der allgemeinen Formel I können auf an sich bekannte Weise hergestellt werden, indem man
a) zur Herstellung von Verbindungen der allgemeinen Formel Ia 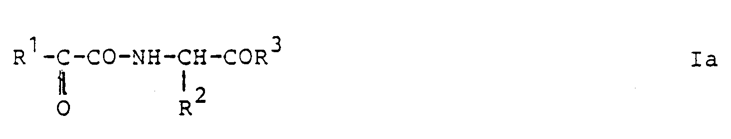 worin R¹ , R² und R³ obige Bedeutung besitzen,
   in Verbindungen der allgemeinen Formel II 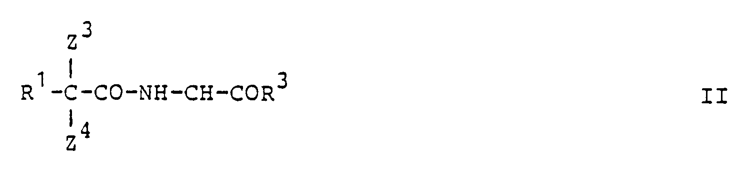 worin R¹ , R² und R³ obige Bedeutung besitzen, und
   Z³ und Z⁴ Alkoxy oder Alkylthio bedeuten oder gemeinsam für Alkylendioxy oder Alkylendithio stehen, oder worin Z³ Alkoxy oder Alkylthio bedeutet und Z⁴ gemeinsam mit Y eine Bindung darstellt,
   die CZ³Z⁴-Gruppierung in eine Ketogruppe überführt oder
b) zur Herstellung von Säuren der allgemeinen Formel Ib 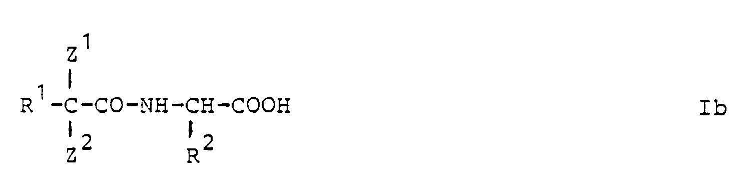 worin R¹ , R² , Z¹ und Z² obige Bedeutung besitzen,
   Ester der allgemeinen Formel Ic 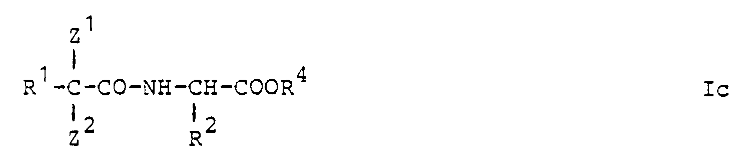 worin R¹ , R² , Z¹ und Z² obige Bedeutung besitzen und R⁴ niederes Alkyl bedeutet,
   hydrolisiert oder
c) zur Herstellung von Verbindungen der allgemeinen Formel Id 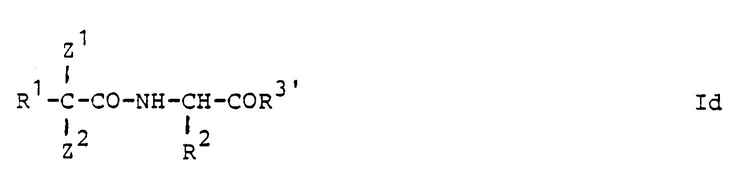 worin R¹ , R² , Z¹ und Z² obige Bedeutung besitzen und R³′ niederes Alkoxy oder eine R⁵′R⁶′-N-Gruppe darstellt, worin R⁵′ und R⁶′ die für R⁵ und R⁶ angegebene Bedeutung mit Ausnahme von Wasserstoff besitzen,
   Verbindungen der allgemeinen Formel III 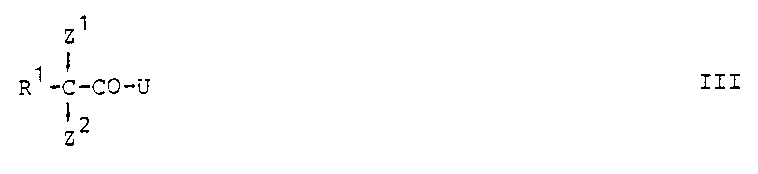 worin R¹ , Z¹ und Z² obige Bedeutung besitzen, und U ein reaktiver Rest ist,
   mit Verbindungen der allgemeinen Formel IV 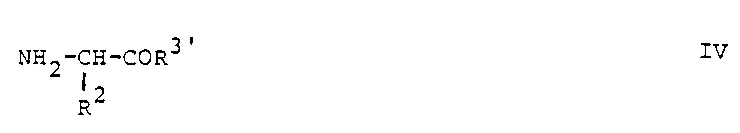 worin R² und R³′ obige Bedeutung besitzen,
   umsetzt oder
d) zur Herstellung von Verbindungen der allgemeinen Formel Ie 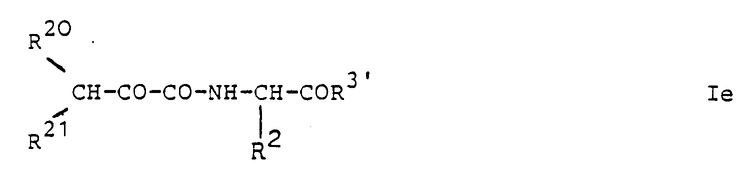 worin R²⁰ , R²¹ , R² und R³′ obige Bedeutung besitzen,
   Verbindungen der allgemeinen Formel V 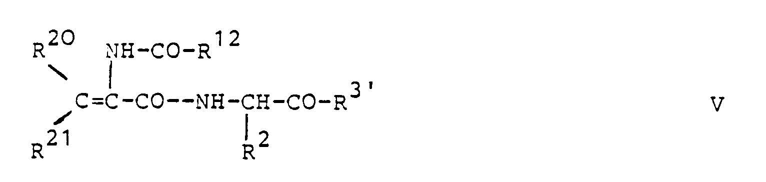 worin R²⁰ , R²¹ ,R² und R³′ obige Bedeutung besitzen und R¹² niederes Alkyl, Phenyl, Trifluormethyl oder Trichlormethyl bedeutet,
   spaltet oder
e) Säuren der allgemeinen Formel Ib oder deren reaktionsfähige Säurederivate mit Aminen der allgemeinen Formel VI 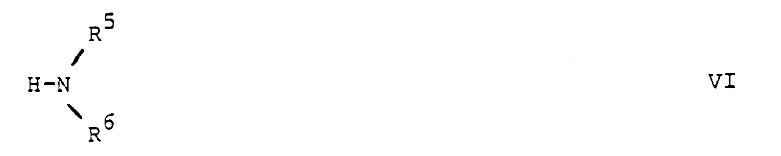 worin R⁵ und R⁶ obige Bedeutung besitzen,
   umsetzt zu Verbindungen der allgemeinen Formel If 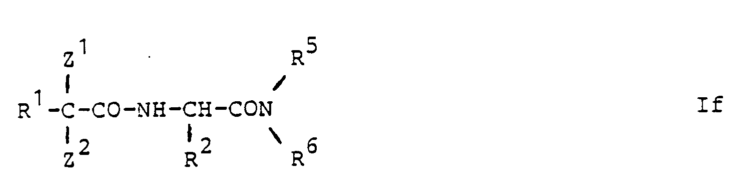 worin R¹ , R² , R⁵ , R⁶ , Z¹ und Z² obige Bedeutung besitzen,
   und gewünschtenfalls Säuren der Formel Ib in ihre Salze mit physiologisch verträglichen Kationen überführt oder Salze der Säuren der Formel Ib in die freien Säuren überführt.

Die Umsetzung der Verbindungen der allgemeinen Formel II zu Verbindungen der Formel Ia gemäß Verfahrensvariante a) kann nach an sich zur hydrolytischen Spaltung von Ketal-und Enolethergruppen üblichen Methoden ausgeführt werden. Die hydrolytische Spaltung kann in einer wässrigen Lösung oder Suspension der Verbindung der Formel II, gegebenenfalls unter Verdünnung mit einem unter den Reaktionsbedingungen inerten Lösungsmittel, unter sauren Reaktionsbedingungen erfolgen. Für die saure Hydrolyse eignen sich wäßrige Lösungen von organischen und anorganischen Säuren. Hierfür verwendbare organische Säuren sind niedere Alkancarbonsäuren wie Ameisensäure, Essigsäure etc, Halogenalkancarbonsäuren wie Chloressigsäure oder organische Sulfonsäuren wie p-Toluolsulfonsäure; verwendbare anorganische Säuren sind Mineralsäuren wie Salzsäure oder Phosphorsäure. Besonders gut werden Oxoketale der Formel II, worin Z³ und Z⁴ für alkoxy oder gemeinsam für Alkylendioxy stehen oder Z³ Alkoxy und Z⁴ gemeinsam mit Y eine Bindung darstellt, in einfacher Weise durch Umsetzung mit einer organischen oder anorganischen Säure in wäßrigem Medium gespalten. Vorzugsweise verwendet man dazu verdünnte bis konzentrierte wässrige Mineralsäuren, insbesondere Salzsäure. Sollen Thioketale der Formel II, worin Z³ und Z⁴ für Alkylthio oder gemeinsam für Alkylendithio stehen oder Z³ Alkylthio und Z⁴ gemeinsam mit Y eine Bindung darstellt, gespalten werden, erweist es sich als günstig, die saure Hydrolyse in Ethern, vorzugsweise in cyclischen Ethern wie Dioxan auszuführen. Darüber hinaus kann die Thioketalgruppe zwar in einfacher Weise durch Umsetzung mit HgO/HgCl₂/H₂O gespalten werden, doch ist dieser Weg für die Gewinnung hochreiner, für pharmazeutische Anwendungen geeigneter Verbindungen von nur geringerer Bedeutung. Die Umsetzung der Ketale der Formel II zu Verbindungen der Formel Ia kann bei Temperaturen zwischen Raumtemperatur und dem Siedepunkt des Lösungsmittels erfolgen, wobei der sich bei der Umsetzung bildende Alkohol gegebenenfalls abdestilliert wird.

Zur Herstellung von Säuren der allgemeinen Formel Ib gemäß Verfahrensvarinate b) können Ester der allgemeinen Formel Ic nach an sich zur Hydrolyse von Estergruppierungen üblichen Methoden im alkalischen oder sauren wäßrigen Medium hydrolysiert werden. Die Hydrolyse kann in einer wäßrigen Lösung oder Suspension der Verbindungen der Formel Ic, gegebenenfalls unter Verdünnung mit einem unter den Reaktionsbedingungen inerten Lösungsmittel in Gegenwart von Säuren oder Alkalien erfolgen. Als Säuren können wäßrige Lösungen von organischen oder anorganischen Säuren verwendet werden. Hierfür geeignete organische Säuren sind niedere Alkancarbonsäuren wie Ameisensäure, Essigsäure etc., Halogenalkancarbonsäuren wie Chloressigsäure oder organischen Sulfonsäuren wie p-Toluolsulfonsäure; geeignete anorganische Säuren sind Mineralsäuren wie Salzsäure oder Phosphorsäure. Als Alkalien können wäßrige Lösungen oder Suspensionen von Oxiden, Hydroxiden oder Carbonaten von Alkali- oder Erdalkalimetallen verwendet werden. Zweckmäßig führt man die Hydrolyse der Ester der Formel Ic unter alkalischen Reaktionsbedingungen unter Verwendung von wäßrigen Alkalihydroxiden aus. Vorzugsweise wird die Hydrolyse mit wäßriger Natronlauge ausgeführt. Vorzugsweise können als Verbindungen Ic die Methoxy-(R⁴ = CH₃) oder Ethoxyester (R⁴ = C₂H₅) eingesetzt werden. Die Umsetzung der Ester der Formel Ic zu Verbindungen der Formel Id kann bei Temperaturen zwischen Raumtemperatur und dem Siedepunkt des Lösungsmittels erfolgen, wobei man gegebenenfalls den sich bei dieser Umsetzung bildenden Alkohol abdestilliert.

Die Herstellung von Verbindungen der allgemeinen Formel Id gemäß Verfahrenssvariante c), kann durch Umsetzung von reaktiven Säurederivaten der Formel III, worin U für einen reaktiven Rest steht, mit Aminen der allgemeinen Formel IV nach an sich in der Peptidchemie zu Bildung von Amid-Gruppierungen durch Aminoacylierung üblichen Methoden ausgeführt werden. Als reaktionsfähige Derivate der Formel III kommen insbesondere Säurehalogenide, vorzugsweise -chloride, Ester und gemischte Anhydride in Frage, z.B. Verbindungen der Formel III, worin die reaktive Gruppe U Halogen, insbesondere Chlor oder Brom, niederes Alkoxy, insbesondere Alkoxy mit 1-4 Kohlenstoffatomen, oder eine Gruppe O-W bedeutet, worin W für eine Niederalkylcarbonyl- oder Niederalkoxycarbonyl-Gruppe oder einen organischen Sulfonsäurerest, insbesondere den Rest einer Niederalkansulfonsäure wie z.B. Methansulfonsäure oder einer aromatischen Sulfonsäure wie Benzolsulfonsäure oder durch niederes Alkyl oder Halogen substituierte Benzolsulfonsäuren, steht. Es ist auch möglich, von der zugrundeliegenden Säure der Formel III (U = OH) des reaktiven Säurederivates selbst auszugehen. Die Säure wird dann erst vor der eigentlichen Umsetzung mit dem Amin der Formel IV nach an sich bekannten Methoden in situ in das reaktionsfähige Säurederivat übergeführt, welches anschließend ohne weitere Isolierung oder Reinigung für die Umsetzung mit dem Amin der Formel IV verwendet wird. Die in situ-Bildung des reaktionen Säurederivates kann zweckmäßigerweise bei Temperaturen von -30 °C bis Raumtemperatur unter Benutzung von Lösungsmitteln wie halogenierten Kohlenwasserstoffen, Ethern, vorzugsweise cyclischen Ethern wie Tetrahydrofuran, und/oder aromatischen Lösungsmitteln durchgeführt werden.

Vorzugsweise verwendet man als Verbindungen der Formel III Säurehalogenide, insbesondere Säurechloride, oder gemischte Säureanhydride, insbesondere aus Umsetzung der zugrundeliegenden Säuren der Formel III (U = OH) mit einem organischen Sulfonsäurechlorid wie Methansulfonsäurechlorid oder mit Chlorameisensäureester erhaltene gemischte Anhydride. In einer bevorzugten Arbeitsweise werden solche Säurederivate eingesetzt, die erst vor der eigentlichen Umsetzung aus der zugrundeliegenden Säure der Formel III (U = OH) in situ hergestellt wurden, und die ohne vorherige Isolierung oder Reinigung direkt mit dem Amin der Formel IV unter Amidbildung zur Reaktion gebracht werden. Die Umsetzung des Amins IV mit dem Säurehalogenid oder -anhydrid der Formel III wird in Gegenwart eines inerten organischen Lösungsmittels beispielsweise eines halogenierten Kohlenwasserstoffes wie Methylenchlorid, eines cyclischen oder offenen Ethers wie Dioxan oder Diethylether, Dimethylformamid, Sulfolan, Tetramethylharnstoff oder Gemischen dieser Lösungsmittel und gegebenenfalls aromatischer Kohlenwasserstoffe wie Benzol oder Toluol durchgeführt. Sofern Säurehalogenide oder -anhydride der Formel III eingesetzt werden, ist es zweckmäßig, die Reaktion in Gegenwart eines säurebindenden Mittels durchzuführen. Als säurebindende Mittel eignen sich anorganische Basen, beispielsweise Alkalimetallcarbonate, oder -hydroxide oder organische Basen, insbesondere tertiäre Niederalkylamine, z.B. Triethylamin oder Pyridine. Statt einer Fremdbase kann auch ein Überschuß des Amins der Formel IV verwendet werden. Im Überschuß eingesetzte organische Basen können gleichzeitig auch als Lösungsmittel dienen. Es kann vorteilhaft sein darüber hinaus katalytische Mengen von basischen Pyridinen wie 4-Dimethylaminopyridin oder 4-Pyrrolidinpyridin zuzusetzen. Vorteilhafterweise wird die Reaktion bei Temperaturen zwischen -30°C und Siedetemperatur des Reaktionsgemisches durchgeführt. Die gewählte Temperatur kann je nach deneingesetzten Ausgangsverbindungen variieren, beispielsweise sind bei Verwendung von Säurehalogeniden oder -anhydriden der Formel III niedrige Temperaturen bis ca. Raumtemperatur, insbesondere Temperaturen von ca. -20 °C bis 0 °C, bevorzugt. Insbesondere erweise es sich als vorteilhaft, eine stark gekühlte Lösung des Säurederivates der Formel III mit einer Lösung des Amins der Formel IV umzusetzen.

Die Verfahrensvariante c) eignet sich insbesondere zur Umsetzung von Verbindungen der allgemeinen Formel III, worin Z¹ und Z² für Alkoxy oder gemeinsam für Alkylendioxy stehen oder Z¹ Alkoxy und Z² gemeinsam mit Y eine Bindung darstellt. Aber auch Ketoverbindungen der Formel III (Z¹ und Z² = gemeinsam Sauerstoff) lassen sich mit Aminen der Formel IV zu Verbindungen der Formel Id umsetzen.

Gemäß Verfahrensvariante d) werden die Verbindungen der allgemeinen Formel V hydrolytisch gespalten. Diese hydrolytische Spaltung der Enamidverbindungen der Formel V kann nach an sich in der Peptidchemie zur Spaltung von Enamidgruppierungen üblichen Methoden ausgeführt werden. Die Hydrolyse der Enamidverbindungen der Formel V erfolgt zweckmäßigerweise in wäßrigem Medium unter sauren Reaktionsbedingungen, gegebenenfalls in Gegenwart eines mit Wasser mischbaren organischen Lösungsmittels; geeignete Lösungsmittel sind z.B. niedere Alkohole, Aceton oder cyclische Ether wie Dioxan oder Furan, vorzugsweise niedere Alkohole wie Methanol oder Ethanol. Zweckmäßig können Verbindungen der Formel V eingesetzt werden, worin R¹² für Trifluormethyl steht. In einfacher Weise kann die Enamidgruppierung durch Behandeln mit einer wässrigen Lösung einer organischen oder anorganischen Säuren gespalten werden. Als organische Säuren eigenen sich niedere Alkancarbonsäuren wie Ameisensäure, Essigsäure etc. oder Halogencarbonsäuren wie Chloressigsäure; als anorganische Säuren eignen sich z.B. Salzsäure oder Phosporsäure. Insbesondere verwendet man wäßrige Mineralsäuren, vorzugsweise Salzsäure. Die Hydrolyse der Enamidverbindugnen kann bei Temperaturen zwischen Raumtemperatur und dem Siedepunkt des Lösungsmittels erfolgen.

Die Umsetzung der Säuren der Formel Ib oder deren reaktionsfähigen Säurederivate mit Aminen der Formel VI gemäß Verfahrensvariante e) kann nach an sich in der Peptidchemie zur Bildung von Amid-Gruppierungen durch Aminoacylierung üblichen Methoden ausgeführt werden, beispielsweise unter den oben für Umsetzung von III mit IV angegebenen Bedingungen. Die Verfahrensvariante e) eignet sich insbesondere zur Umsetzung von Verbindungen der allgemeinen Formel Ib, worin Z¹ und Z² für Alkoxy oder gemeinsam für Alkylendioxy stehen oder Z¹ Alkoxy und Z² gemeinsam mit Y eine Bindung darstellt. Aber auch Ketoverbindungen der Formel Ib (Z¹ und Z² = gemeinsam Sauerstoff) lassen sich mit Aminen der Formel VI zu Verbindungen der Formel If umsetzen.

Falls in den eingesetzten Aminen der Formel VI R⁵ für Wasserstoff und R⁶ für den Desaminorest einer biogenen α-Aminocarbonsäure stehen, ist es zweckmäßig, die darin enthaltene freie Carboxygruppe vor den obigen Umsetzungen in an sich bekannter Weise mit einer Schutzgruppe zu versehen, welche anschließend leicht wieder abspaltbar ist. Geeignete Schutzgruppen sind z.B. bekannt aus E. McOmie, "Protective Groups in Organic Chemistry", Plenum Press 1971. Beispielsweise eignen sich zum Schutz von in Aminocarbonsäuren vorhandenen Carboxygruppen Estergruppen wie z.B. Methylester, Benzylester, p-Nitrophenylester etc.

Sofern der Desaminorest der Aminocarbonsäure neben der Carboxygruppe weitere funktionelle Gruppen enthält, so können auch diese gegebenenfalls während der obigen Umsetzungen mit Schutzgruppen versehen sein.

Die Verbindungen der Formel I können auf an sich bekannte Weise aus dem Reaktionsgemisch isoliert und gereinigt werden. Salze von Säuren der Formel I können in üblicher Weise in die freien Säuren überführt werden und diese gewünschtenfalls in bekannter Weise in pharmakologisch verträgliche Salze dieser Säuren überführt werden . Die pharmakologisch verträglichen Salze von Säuren der Formel I mit Metallkationen und Ammoniumionen der Formel C werden nach an sich zur Salzbildung üblichen Methoden hergestellt.

Salze mit Metallkationen erhält man beispielsweise, indem man die Säuren der Formel I in einem mit Wasser mischbaren organischen Lösungsmittel, insbesondere in niederen Alkoholen wie Methanol oder Ethanol, löst und mit festem, pulverisiertem Metallhydroxid oder mit einer Lösung oder Suspension des Oxids oder Hydroxids des Metallkations in Wasser umsetzt und anschließend das entsprechende Metallsalz der Säure in an sich bekannter Weise isoliert und reinigt. Beispielsweise kristallisieren einige Salze der Säuren der Formel I mit Metallkationen bei Raumtemperatur bereits aus der Reaktionslösung, wobei die Kristallisation durch zusätzliche Abkühlung bis ca. 4 °C vervollständigt werden kann; andere Salze der säuren der Formel I mit Metallkationen können durch Zusatz geeigneter Lösungsmittel, z.B. einerseits durch mit Wasser mischbare organische Lösungsmittel, wie Aceton oder andere Ketone sowie Essig ester, oder Kohlenwasserstoffe wie Petrolether oder Hexan aus der Reaktionslösung, ggf. auch unter zusätzlicher Kühlung, ausgefällt werden.

Salze von Säuren der Formel I mit Ammoniumionen der Formel C erhält man beispielsweise, indem man die Säure I in einem mit Wasser mischbaren organischen Lösungsmittel, insbesondere in niederen Alkoholen, Aceton oder Essigester, löst und ein der Ammoniumgruppe C zugrundeliegendes Amin, oder ein die Ammoniumgruppe C enthaltendes Ammoniumsalz gelöst in organischen Lösungsmitteln wie Halogenkohlenwasserstoffen, insbesondere Methylenchlorid, oder Kohlenwasserstoffen, insbesondere Hexan, zutropft. Sind insbesondere Salze der Säuren der Formel I mit Kationen von basischen Aminosäuren (R⁸ , R⁹ und R¹⁰ = Wasserstoff und R¹¹ = Desaminorest der basischen Aminosäure) erwünscht, so können diese beispielsweise erhalten werden, indem man die Säure I in einem mit Wasser mischbaren organischen Lösungsmittel, insbesondere niedere Alkohole wie Methanol, löst und mit einer Lösung der basischen Aminosäure in niedrigen Alkoholen, insbesondere Methanol, umsetzt und anschließend das entsprechende Salz der Säure mit der basischen Aminosäure in an sich bekannter Weise isoliert und reinigt. Gegebenenfalls kristallisieren die Salze der Säuren der Formel I mit basischen Aminosäuren bei Raumtemperatur bereits aus der Reaktionslösung; andererseits können Salze der Säuren der Formel I mit basischen Aminosäuren auch durch Zusatz geeigneter Lösungsmittel, z.B. durch mit Wasser mischbare Lösungsmittel wie Aceton oder anderer Ketone sowie Essigester, ggf. auch unter zusätzlicher Kühlung aus der Reaktionslösung ausgefällt werden.

Die Ausgangsstoffe für die Herstellung der Verbindungen der Formel I können nach an sich bekannten Verfahren erhalten werden.

Die in der Verfahrensvariante a) einzusetzenden Ketal- und Enoletherverbindungen der Formel II können durch Umsetzung von Säuren oder Säurederivaten der Formel VII 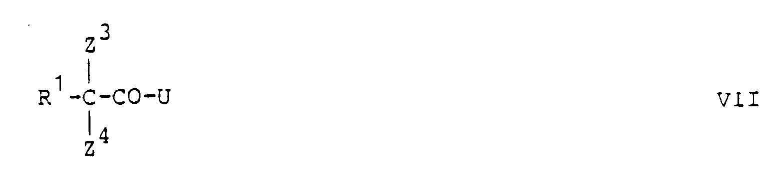
worin Z³ und Z⁴ die obige Bedeutung besitzen und U für Hydroxy oder einen reaktiven Rest steht, mit Aminen der allgemeinen Formel IV nach an sich in der Peptidchemie zur Bildung von Amid-Gruppierungen durch Aminoacylierung üblichen Methoden erhalten werden, beispielsweise unter den oben für Umsetzungen von III mit IV angegebenen Bedingungen.

Die Verbindungen der Formel VII können durch Ketalisierung der zugrunde liegenden α-Ketosäureverbindungen der allgemeinen Formel IIIa 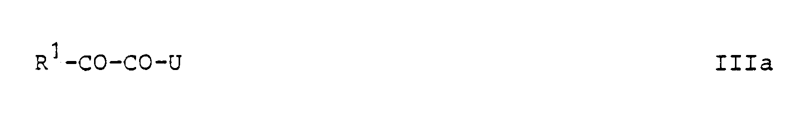
worin R¹ und U obige Bedeutung besitzen, nach an sich üblichen Methoden hergestellt werden. Beispielsweise führt man die Ketalisierung unter Katalyse von wasserfreien organischen oder anorganischen Säuren durch Umsetzung von α-Ketosäuren oder deren Derivaten mit Alkoholen, Alkylendiolen, Thiolen oder Alkylendithiolen aus. Das bei der Ketalisierung mit Alkoholen oder Alkylendiolen gebildete Reaktionswasser wird gegebenenfalls durch wasserbindende Mittel abgefangen, z.B. mit Dialkylsulfiten bei der Herstellung von Dialkyl-oxoketalen. Man kann das gebildete Reaktionswasser auch durch azeotrope Destillation entfernen. Anstelle der Alkohole können als Ketalisierungsmittel auch die entsprechenden Orthoameisensäuretrialkylester, z.B. Orthoameisensäuretrimethyl- oder -triethylester eingesetzt werden.

Die Amine der Formel IV sind bekannt oder können auf an sich bekannte Weise hergestellt werden, indem man die zugrunde liegende Aminosäure nach an sich üblichen Methoden zur Ester- bzw. Amidbildung umsetzt.

Die in der Verfahrensvariante c) einzusetzenden Verbindungen der Formel III können nach an sich üblichen Methoden erhalten werden. Die Ketosäuren der Formel IIIa (U = OH) sind bekannt oder können auf an sich bekannte Weise erhalten werden, z.B. indem man die zugrundeliegende Aminosäure der Formel XIIIa, 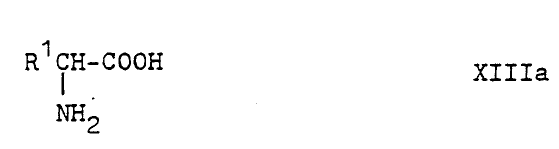
worin R¹ die obige Bedeutung besitzt, zunächst mit Trifluoressigsäureanhydrid (Formel XIV, R¹² = CF₃) zum 4-substituierten 2-Trifluormethyl-5-oxazolon der Formel XIa, 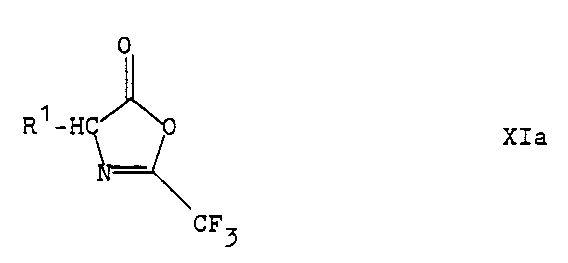
worin R¹ die obige Bedeutung besitzt, unter Erwärmung entweder lösungsmittelfrei oder auch in Gegenwart von Lösungsmitteln wie z.B. Methylenchlorid umsetzt. Das so erhaltene Oxazolon der Formel XIa kann anschließend durch basische Hydrolyse, bei der gleichzeitig eine intramolekulare Oxidation-Reduktion-Reaktion stattfindet, in die Ketosäure der Formel IIIa überführt werden; für diese hydrolytische Umsetzung sind z.B. Alkalien geeignet wie Natrium- bzw. Kaliumhydroxid. Im Falle der Umwandlung der Aminosäure Isoleucin (R¹ = -CH(CH₃)CH₂CH₃ in XIIIa) in die entsprechende Ketosäure erweist es sich als günstig, die Hydrolyse des entsprechenden Oxazolons (R¹ = -CH(CH₃)CH₂CH₃ in Formel XIa) in einem Puffer bei ca. pH = 6,8 durchzuführen, um eine Racemisierung des im Isoleucin-Rest R¹ = -CH(CH₃)CH₂CH₃ vorhandenen asymmetrischen C-Atoms zu vermeiden.

Die Säuren der Formel IIIa können auf an sich bekannte Weise in ihre reaktionsfähigen Derivate überführt werden. Ketale und Enolketale der Formel III (Z¹ und Z² = je Alkoxy oder gemeinsam Alkylendioxy oder Z¹ Alkoxy und Z² gemeinsam mit Y eine Bindung) können durch Ketalisierung der zugrundeliegenden Ketoverbindungen der Formel IIIa mit Alkoholen oder Alkylendiolen nach an sich üblichen Methoden hergestellt werden, beispielsweise unter den für die Herstellung von VII angegebenen Bedingungen.

Die in der Verfahrensvariante d) einzusetzenden Ausgangsverbindungen der Formel V können durch Umsetzung von substituierten, ungesättigten Oxazolonen der allgemeinen Formel VIII 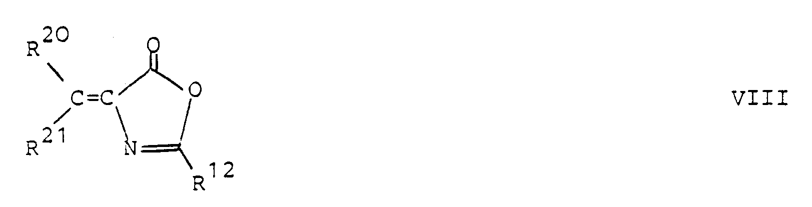
worin R²⁰ , R²¹ und R¹² die obige Bedeutung besitzen, mit Aminen der allgemeinen Formel IV gewonnen werden. Die Umsetzung von VIII mit IV erfolgt unter an sich in der Peptidchemie üblichen Bedingungen, beispielsweise unter den oben für Umsetzungen von III mit IV angegebenen Bedingungen.

Für die Herstellung der Oxazolone VIII bieten sich zwei Wege an. Der erste Weg zur Herstellung von ungesättigten Oxazolonen der Formel VIII geht von Ketoverbindungen der Formel IX 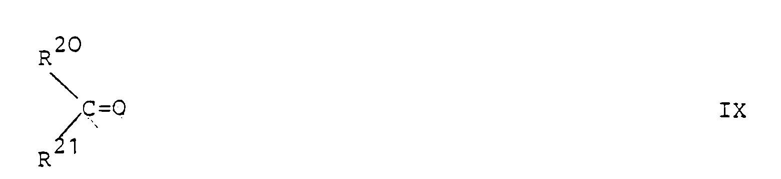
aus, worin R²⁰ und R²¹ obige bedeutung besitzen. Diese werden mit N-Acylglycinderivaten der Formel X 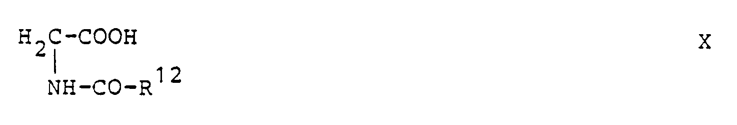
worin R¹² die obige Bedeutung besitzt, in an sich bekannter Weise (Erlenmeyer-Synthese) kondensiert. Hierzu wird beispielsweise IX mit X in einem geeigneten Lösungsmittel wie z.B. Methanol unter Zusatz von tertiären Basen wie Pyridin oder in Gegenwart von Natriumacetat und Acetanhydrid umgesetzt. Die Kondensation kann bei Temperaturen zwischen Raumtemperatur und dem Siedepunkt des Lösungsmittels erfolgen. Der zweite Weg zur Herstellung von ungesättigten Oxazolonen der Formel VIII geht in an sich bekannter Weise von substituierten, gesättigten Oxazolonen der Formel XI 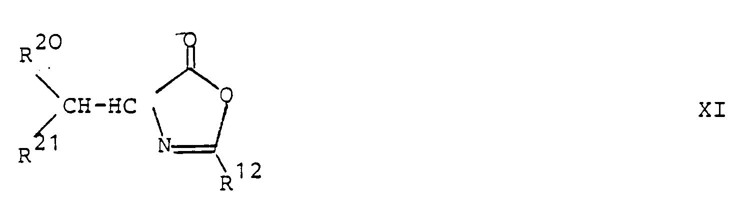
aus, worin R²⁰ , R²¹ und R¹² die obige Bedeutung besitzen. Diese werden zunächst zu Verbindungen der allgemeinen Formel XII 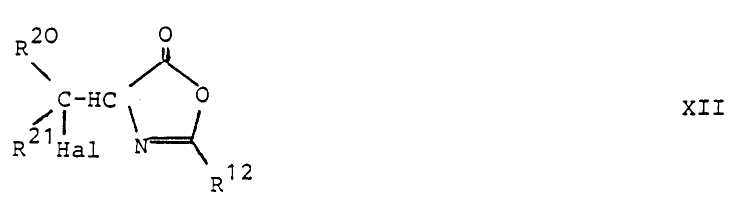
worin R²⁰ , R²¹ und R¹² obige Bedeutung besitzen und Hal für Halogen, vorzugsweise Brom steht, halogeniert, vorzugsweise bromiert. Die Halogenierung der Verbindungen der Formel XI erfolgt unter an sich bekannten Bedingungen, in dem man XI in einem unter den Reaktionsbedingungen inerten Lösungsmittel, insbesondere 1,2-Dichlorethan, mit dem Halogen, vorzugsweise mit Brom, zu XII umsetzt. Anschließend werden die halogenierten Derivate XII durch Behandlung mit einer organischen Base, vorzugsweise Triethylamin, zu den ungesättigten Oxazolonen der Formel VIII dehydrohalogeniert. XII kann hierzu in an sich bekannter Weise in einem unter den Reaktionsbedingungen inerten Lösungsmittel wie Ether, beispielsweise cyclische Ether wie Tetrahydrofuran durch Behandeln mit einer tertiären organischen Base, vorzugsweise durch Behandeln mit der tertiären organischen Base Triethylamin, zu VIII umgesetzt werden.

Die Oxazolone der Formel XI können in an sich bekannter Weise hergestellt werden, indem man z.B. eine α-Aminocarbonsäure der Formel XIII 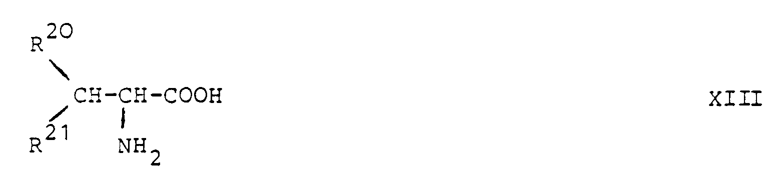
worin R²⁰ und R²¹ die obige Bedeutung besitzen, unter Erhitzen mit einem Säureanhydrid der Formel XIV 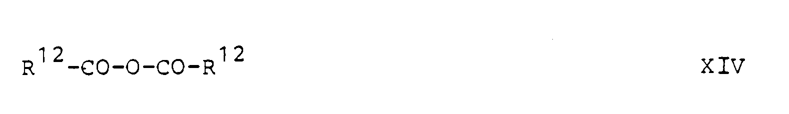
worin R¹² die obige Bedeutung besitzt, gegebenenfalls unter Verdünnung mit einem unter den Reaktionsbedingungen inerten Lösungsmittel, reagieren läßt; oder indem man z.B. eine N-acylierte α-Aminocarbonsäure der Formel XV 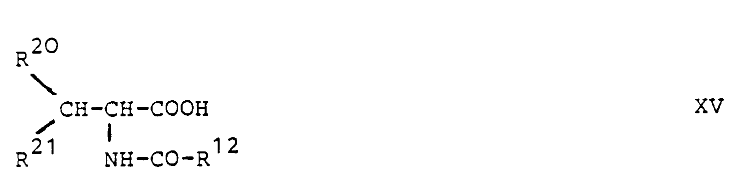
worin R²⁰ , R²¹ und R¹² die obige Bedeutung besitzen, mit einem anorganischen Säurehalogenid wie Phosphortrichlorid, Phosphorpentachlorid, Phosphoroxichlorid oder Thionylchlorid, vorzugsweise Phosphoroxichlorid, in Gegenwart einer tertiären organischen Base wie z.B. Pyridin, gegebenenfalls unter Verdünnung mit einem unter den Reaktionsbedingungen inerten Lösungsmittel, umsetzt; oder in dem man z.B. eine N-acylierte α-Aminocarbonsäure der Formel XV mit Chlorameisensäure-isopropylmethylester in Gegenwart einer tertiären organischen Base wie Triethylamin, gegebenenfalls unter Verdünnung mit einem unter den Reaktionsbedingungen inerten Lösungsmittel, umsetzt.

In einer besonderen Variante zur Herstellung von Enamidverbindungen der Formel V durch Umsetzung von ungesättigten, substituierten Oxazolonen der Formel VIII mit Aminen der Formel IV kann das ungesättigte Oxazolon der Formel VIII auch in situ aus dem bromierten Oxazolon der Formel XII gebildet werden. Hierzu wird XII direkt in eine Lösung des Amins IV und einer tertiären organischen Base wie z.B. Triethylamin gegeben.

Die nachfolgenden Beispiele sollen die Herstellung der neuen Verbindungen der Formel I näher erläutern, jedoch den Umfang der Erfindung in keiner Weise beschränken.

Die Strukturen der neuen Verbindungen wurden durch Elementaranalyse und spektroskopische Untersuchungen, insbesondere durch Analyse der NMR-, Massen- oder IR-Spektren gesichert.

### Beispiel 1; Ethylenketal des Ketoleucylleucin-methylester

a) 10,0 g Ketoleucin wurden mit 16,7 g Ethylenglykol unter Zusatz von 0,15 g p-Toluolsulfonsäure in 160 ml Toluol 16 h in der Siedehitze umgesetzt, wobei gebildetes Reaktionswasser ständig aus dem Reaktionsgemisch abdestilliert und über einen Wasserabscheider abgetrennt wurde. Nach beendeter Reaktion wurde das Reaktionsgemisch mit Wasser gewaschen und über Natriumsulfat getrocknet. Anschließend entfernte man das Lösungsmittel Toluol unter vermindertem Druck. Als Rückstand erhielt man 16,8 g des Ethylenketals von Ketoleucylglykolester.
b) Eine Lösung von 16,0 g des unter a) erhaltenen Ethylenketals von Ketoleucylglykolester in 100 ml Methanol wurde nach Zugabe von 91,5 ml einer 2 N Natriumhydroxid-Lösung 1 h bei Raumtemperatur gerührt. Anschließend entfernte man das Lösungsmittel Methanol unter vermindertem Druck und stellte die verbleibende wässrige Lösung mit konzentrierter Salzsäure auf den pH-Wert 1 ein. Nach 15 minütigem Rühren der sauren Lösung wurde die gebildete freie Säure des Ethylenketals von Ketoleucin mit Methylenchlorid extrahiert und das Methylenchloridextrakt über Natriumsulfat getrocknet. Durch Entfernung des Lösungsmittels Methylenchlorid unter vermindertem Druck erhielt man 11,4 g des Ethylenketals von Ketoleucin.
c) 11,4 g des unter b) erhaltenen Ethylenketals von Ketoleucin und 8,7 g Dimethylaminopyridin wurden in 600 ml Tetrahydrofuran gelöst und nach Zugabe von 13,2 g Triethylamin auf -20 °C abgekühlt. Anschließend wurde bei dieser Temperatur eine Lösung von 8,2 g Mesylchlorid (Methylsulfonsäurechlorid) in 25 ml Tetrahydrofuran zugetropft, wobei man eine Lösung eines an der Säuregruppe aktivierten Derivates des Ethylenketals von Ketoleucin erhielt. Dieser Lösung wurden zur Peptidbildung 11,8 g Leucinmethylester-hydrochlorid zugegeben, wonach man das Reaktionsgemisch auf Raumtemperatur erwärmte.
   Zur Isolierung des bei dieser Reaktion gebildeten Peptidderivates wurden gebildete Niederschläge abgesaugt und das Lösungsmittel unter vermindertem Druck entfernt. Der verbliebene ölige Rückstand wurde in Methylenchlorid gelöst, mit Wasser gewaschen und die erhaltene Methylenchloridlösung dann über Natriumsulfat getrocknet. Nach Entfernung des Methylenchlorids unter vermindertem Druck verblieb ein öliges Produkt, welches säulenchromatographisch (Kieselgel; Laufmittel Hexan/Essigester 2 : 1) gereinigt wurde. Es wurden 18,4 g des Ethylenketals von Ketoleucylleucin-methylester in Form eines gelben Öles isoliert.
   Die hergestellte Verbindung wies im IR-Spektrum (Film) Banden bei folgenden Wellenzahlen (in cm⁻¹) auf: 1740, 1680, 1520.

In analoger Verfahrensweise zum Beispiel 1 wurden die in Tabelle 1 angegebenen Ethylenketale hergestellt.

**Tabelle 1**

| Beispiel | Ethylenketal von | IR-Daten |
|---|---|---|
| 2 | Ketoleu-Val-OMe | 1740 |
| | | 1720 |
| | | 1670 |
| | | 1520 |
| 3 | Ketoval-Leu-OMe | 1740 |
| | | 1680 |
| | | 1515 |
| 4 | Ketoval-Val-OMe | 1740 |
| | | 1650 |
| | | 1530 |

### Beispiel 5: Ethylenketal des Ketoleucylleucins

Zu einer Lösung von 18,4 g des in Beispiel 1 erhaltenen Ethylenketals von Ketoleucylleucin-methylester in 50 ml Methanol wurden 240 mmol Natriumhydroxid in Form einer wäßrigen 2 N Natriumhydroxid-Lösung zugegeben und die Reaktionslösung 2 h bei Raumtemperatur gerührt. Nach Entfernung des Lösungsmittels unter vermindertem Druck erhielt man die rohe Titelverbindung in Form ihres Natriumsalzes.

Das so gewonnene rohe Produkt kann zwar in die freie Säure überführt und anschließend gereinigt werden; es kann aber auch direkt für weitere Umsetzungen verwendet werden.

### Beispiel 6: Ketoleucylleucin

Das in Beispiel 5 erhaltene rohe Produkt wurde mit konzentrierter Salzsäure auf den pH-Wert 1 eingestellt und das so erhaltene Gemisch 15 Minuten gerührt. Anschließend wurde mit Methylenchlorid extrahiert und der Methylenchloridextrakt über Natriumsulfat getrocknet. Nach Entfernung des Lösungsmittels Methylenchlorid unter vermindertem Druck und Reinigung des Rückstandes durch Säulenchromatographie (Kieselgel; Laufmittel Hexan/Essigester 1 : 1) erhielt man 12,2 g Ketoleucylleucin in Form der freien Säure als hellgelbes Öl.

Die hergestellte Verbindung wies im IR-Spektrum (Film) Banden bei folgenden Wellenzahlen (in cm⁻¹) auf: 1720, 1710, 1670, 1530. Im ¹³C-NMR-Spektrum wurden folgende Resonanzen gefunden (in ppm): 201,5; 160,0; 57,2; 175,5.

In analoger Verfahrensweise zu den Beispielen 5 und 6 wurden die in der nachfolgenden Tabelle 2 wiedergegebenen freien Ketodipeptidsäuren erhalten.

**Tabelle 2**

| Beispiel | Produkt | IR-Daten (cm⁻¹) | ¹³C-NMR-Daten (ppm) | Bemerkungen |
|---|---|---|---|---|
| 7 | Ketoleu-Val | 1733 | 197,9 | Schmelzpunkt 174-178 °C |
| | | 1718 | 160,3 | |
| | | 1661 | 57,2 | |
| | | 1539 | 175,7 | |
| 8 | Ketoval-Leu | 1720 | | |
| | | 1710 | | |
| | | 1650 | | |
| | | 1525 | | |
| 9 | Ketoval-Val | 1710 | 201,5 | |
| | | 1660 | 160,0 | |
| | | 1550 | 57,2 | |
| | | | 175,5 | |

### Beispiel 10: Ketovalylleucin-methylester

a) Eine Lösung von 20,0 g des Calciumsalzes von Ketovalin in 100 ml destilliertem Wasser wurde mit 44 ml konzentrierter Salzsäure versetzt und 20 Minuten gerührt. Anschließend wurde mit Dichlormethan extrahiert und die gesammelten organischen Phasen über Natriumsulfat getrocknet. Nach Entfernung des Lösungsmittels unter vermindertem Druck verblieben 13,1 g Ketovalin in Form der freien Säure.
b) Eine Lösung von 13,0 g des unter a) erhaltenen Ketovalins in 100 ml Tetrahydrofuran wurde mit 15,1 g Dimethylaminopyridin versetzt. Unter Rühren wurden 22,7 g Triethylamin zugetropft und das Reaktionsgegemisch auf - 20 °C abgekühlt. Anschließend wurde eine Lösung von 14,1 g Mesylchlorid in 50 ml Tetrahydrofuran zugetropft und weitere 20 Minuten gerührt. Dann wurde eine Lösung von 20,4 g Leucinmethylester-hydrochlorid in 150 ml Dichlormethan zugetropft und wiederum 20 Minuten gerührt. Danach ließ man langsam auf Raumtemperatur erwärmen, trennte ungelöste Bestandteile durch Filtration ab und entfernte aus dem Filtrat das Lösungsmittel unter vermindertem Druck. Der Rückstand wurde mit destilliertem Wasser aufgenommen und mit Dichlormethan extrahiert. Die gesammelten organischen Phasen wurden danach über Natriumsulfat getrocknet und nach Filtration das Lösungsmittel unter vermindertem Druck entfernt. Es verblieben 14,7 g der Titelverbindung als gelbes Öl.

Die hergestellte Verbindung wies im IR-Spektrum (Film) Banden bei folgenden Wellenzahlen (in cm⁻¹) auf: 1740, 1720, 1685, 1520.

### Beispiel 11: Ketovalylleucin

Eine Lösung von 22,1 g des in Beispiel 10 erhaltenen Ketovalylleucin-methylesters in 200 ml Methanol wurde mit 113,5 ml einer 2 N Natriumhydroxidlösung versetzt und 1 h gerührt. Anschließend wurde das Lösungsmittel Methanol unter vermindertem Druck entfernt und die erhaltene wäßrige Lösung mit gesättigter Ammoniumchlorid-Lösung gepuffert. Nach Einstellung des Gemisches mit 6 N Salzsäure auf den pH-Wert 2 wurde mit Dichlormethan extrahiert. Die gesammelten organischen Phasen wurden über Natriumsulfat getrocknet und nach Filtration das Lösungsmittel unter vermindertem Druck entfernt. Man erhielt 15,0 g der Titelverbindung in Form eines Öles. Die Verbindung erwies sich in ihren Stoffeigenschaften mit dem nach Beispiel 8 erhaltenen Produkt als identisch.

### Beispiel 12: Ketoisoleucylleucin

In analoger Verfahrensweise zu den Beispielen 10 und 11 wurde das Ketoisoleucylleucin hergestellt. Man erhielt die Titelverbindung in Form eines Öles.

Die hergestellte Verbindung wies im IR-Spektrum (Film) Banden bei folgenden Wellenzahlen (in an⁻¹) auf: 1725, 1710, 1680, 1540.

In analoger Verfahrensweise zu den Beispielen 10 und 11 wurden auch Ketoleucylleucin, Ketoleucylvalin und Ketovalylvalin hergestellt; diese Verbindungen erwiesen sich in ihren Stoffeigenschaften mit dem jeweils entsprechenden, nach Beispiel 6, 7 oder respektive 9 erhaltenen Produkt als identisch.

### Beispiel 13: Ketovalylvalin-methylester

a) Zu einer Lösung von 46,4 g Valinmethylester-hydrochlorid in 500 ml Tetrahydrofuran wurden 314 mmol Triethylamin gegeben und danach 15 Minuten gerührt. Anschließend wurde eine Lösung von 73,9 g α-Brom-oxazolon in 750 ml Tetrahydrofuran zugegeben und danach 24 h bei Raumtemperatur gerührt. Anschließend wurde das Lösungsmittel unter vermindertem Druck entfernt, der Rückstand mit Essigsäureethylester aufgenommen, die Lösung zweimal mit Wasser und einmal mit verdünnter Salzsäure gewaschen. Anschließend wurde die Essigsäureethylesterlösung über Natriumsulfat getrocknet und dann das Lösungsmittel unter vermindertem Druck entfernt. Nach Umkristallisation des verbliebenen Rückstandes aus Methanol/Wasser erhielt man 95,4 g des N-Trifluoracetyl-dehydrovalylvalin-methylesters als weiß-gelbliche Kristalle mit einem Schmelzpunkt von 152 - 155 °C.
b) Eine Lösung von 10,0 g des unter a) erhaltenen N-Trifluoracetyl-dehydrovalylvalin-methylesters in 80 ml Methanol wurden mit 70 ml 4 N Salzsäure versetzt und 3 h unter Rückfluß erhitzt. Danach wurde das Lösungsmittel unter vermindertem Druck entfernt, der verbliebene Rückstand mit verdünnter Natronlauge neutralisiert und mit Dichlormethan extrahiert. Der Dichlormethan-Extrakt wurde über Natriumsulfat getrocknet. Nach Entfernung des Lösungsmittels Dichlormethan unter vermindertem Druck erhielt man 6,5 g des Ketovalylvalinmethylesters in Form eines gelben Öles.
   Die hergestellte Verbindung wies im IR-Spektrum (Film) Banden bei folgenden Wellenzahlen (in cm⁻¹) auf: 1740, 1720, 1680, 1510.

In analoger Verfahrensweise zum Beispiel 13 wurden die in der nachfolgenden Tabelle 3 wiedergegebenen Verbindungen hergestellt.

**Tabelle 3**

| Beispiel | Produkt | IR-Daten (cm⁻¹) | ¹³C-NMR-Daten (ppm) | Bemerkungen |
|---|---|---|---|---|
| 14 | Ketoval-Leu-OMe | 1740 | | Stoffeigenschaften sind mit dem nach Beispiel 10 erhaltenen Produkt identisch |
| | | 1720 | | |
| | | 1685 | | |
| | | 1520 | | |
| 15 | Ketoleu-Leu-OMe | 1740 | 198,2 | |
| | | 1715 | 160,0 | |
| | | 1660 | 51,0 | |
| | | 1550 | 170,7 | |

### Beispiel 16: Diethylamid des Ketoleucylleucins

Eine Lösung von 5,8 g des in Beispiel 15 erhaltenen Ketoleucylleucin-methylesters und 1 g Ammoniumchlorid in 20 ml Diethylamin wurde 1,5 h unter Rückfluß erhitzt. Danach wurde die Reaktionsmischung mit Wasser/Methylenchlorid aufgenommen, die organische Phase abgetrennt und zweimal mit Wasser, sowie nachfolgend zweimal mit 0,1 N Salzsäure gewaschen und dann über Natriumsulfat getrocknet. Nach Entfernen des Lösungsmittels unter vermindertem Druck erhielt man 3,9 g der Titelverbindung als gelbes Öl.

Die Hergestellte Verbindung wies im IR-Spektrum (Film) Banden bei folgenden Wellenzahlen (in cm⁻¹) auf: 1720, 1660, 1650, 1540.

### Beispiel 17: Calciumsalz des Ketoleucylleucins

Zu einer Lösung von 19,1 g des in Beispiel 2 erhaltenen Ketoleucylleucins in 100 ml Methanol wurden 2,9 g Calciumhydroxid zugegeben und das Gemisch kurz unter Rückfluß erhitzt. Aus der abgekühlten Reaktionslösung kristallisierten nach Zugabe von Essigester 15 g des Calciumsalzes von Ketoleucylleucin in Form eines farblosen kristallinen Feststoffes mit einem Schmelzpunkt von 236 bis 237 °C.

Die hergestellte Verbindung wies im ¹³C-NMR-Spektrum folgende Resonanzen auf (in ppm): 198,8; 160,3; 52,9; 180,1.

### Beispiel 18: Calciumsalz des Ketoisoleucylleucins

In analoger Verfahrensweise zum Beispiel 17 wurde das im Beispiel 12 erhaltene Ketoisoleucylleucin in das Calciumsalz überführt. Man erhielt die kristalline Titelverbindung mit einem Schmelzpunkt von 232 - 236 °C.

Die hergestellte Verbindung wies im IR-Spektrum (KBr) Banden bei folgenden Wellenzahlen (in cm⁻¹) auf: 1715, 1650, 1550, 1405.

### Beispiel 19: Magnesiumsalz des Ketoleucylleucins

Zu einer Lösung von 28,5 g des in Beispiel 2 erhaltenen Ketoleucylleucins in 300 ml Methanol wurde eine Suspension von 3,4 g Magnesiumhydroxid in 150 ml Wasser zugegeben und das Gemisch 2 h unter Rückfluß erhitzt. Nach langsamer Abkühlung auf Raumtemperatur wurde das Gemisch noch 1 h unter Kühlung mittels eines Eisbades gerührt. Das kristallisierte farblose Produkt wurde abgesaugt, mit Wasser gewaschen und anschließend getrocknet. Man erhielt 17,6 g der Titelverbindung mit einem Schmelzpunkt von 271 °C.

Die hergestellte Verbindung wies in ¹³C-NMR-Spektrum folgende Resonanzen auf (in ppm): 199,3; 161,9; 54,2; 179,1.

### Beispiel 20: Natriumsalz des Ketoleucylleucins

Eine Lösung von 8,3 g des in Beispiel 2 erhaltenen Ketoleucylleucins in 150 ml Ethanol wurde mit wässriger 1 N Natriumhydroxidlösung auf den pH-Wert 7 eingestellt. Anschließend wurde bis zur einsetzenden Trübung der Lösung Petrolether zugegeben und das Gemisch noch 2 h unter Kühlung mittels eines Eisbades gerührt. Das ausgefallene Produkt wurde abgesaugt, mit Diethylether gewaschen und über Phosphorpentoxid im Vakuum getrocknet. Man erhielt 4,4 g der stark hygroskopischen Titelverbindung mit einem Schmelzpunkt von größer 200 °C.

Die hergestellte Verbindung wies im ¹³C-NMR-Spektrum folgende Resonanzen auf (in ppm): 200,2; 162,0; 53,9; 178,7.

### Beispiel 21: Bisornithat des Ketoleucylleucins

Zu einer Lösung von 28,9 g des in Beispiel 2 erhaltenen Ketoleucylleucins in 300 ml Methanol wurde eine Lösung von 16,2 g der basischen Aminosäure Ornithin in 200 ml Methanol zugegeben und das Gemisch kurz unter Rückfluß erhitzt. Anschließend wurde in der Siedehitze mit Essigester bis zur beginnenden Trübung der Lösung versetzt. Nach langsmer Abkühlung auf Raumtemperatur wurde noch 1 h unter Kühlung mittels eines Eisbades gerührt. Das ausgefallene Produkt wurde abgesaugt, mit Essigester gewaschen und anschließend getrocknet. Man erhielt 26,6 g der Titelverbindung, die Ketoleucylleucin und Ornithin im Molverhältnis von 1:2 enthielt, in Form eines farblosen kristallinen Salzes mit einem Schmelzpunkt von 134-139 °C.

Die Verbindung wies im ¹³C-NMR-Spektrum folgende Resonanzen auf (in ppm): 200,8; 162,5; 54,5; 179,6.

In analoger Verfahrensweise zum Beispiel 21 wurden die in der nachfolgenden Tabelle 4 wiedergegebenen kristallinen Salze von Ketodipeptiden mit der basischen Aminosäure Lysin hergestellt.

**Tabelle 4**

| Beispiel | Lysinat von | IR-Daten (cm⁻¹) | Schmelzpunkt | Molverhältnis Ketodipeptid zu Lysin |
|---|---|---|---|---|
| 22 | Ketoleu-Leu | 1735 | 167 °C | 1 : 1,25 |
| | | 1720 | | |
| | | 1675 | | |
| | | 1520 | | |
| 23 | Ketoval-Val | 3400 | 179-181 °C | 1 : 1 |
| | | 1720 | | |
| | | 1710 | | |
| | | 1680 | | |
| | | 1510 | | |
| | | 1400 | | |

### Beispiel 24: Infusionslösung als Supplement für parenterale Ernährungsregime

Zur Herstellung einer parenteral applizierbaren Lösung wurden Ketoisoleucylleucin in Form des Calcium- oder Ornithinsalzes und Ketovalylvalin in Form des Lysinsalzes unter Rühren in destilliertem Wasser (für Injektionszwecke) gelöst, wobei durch Stickstoffbegasung für weitgehenden Ausschluß von Luftsauerstoff gesorgt wurde. Zur Abscheidung von Partikeln und zur Keimzahlreduzierung wurde die Lösung über eine Filterkaskade mit einem Endfilter von 0,2 µm Porendurchmesser gepumpt. Die eingesetzte Menge der hier verwendeten Salze von Ketoisoleucylleucin und Ketovalylvalin was jeweils so bemessen, daß die fertige Lösung die folgenden, auf die zugrundeliegenden freien Ketodipeptidsäuren der eingesetzten Salze berechneten Konzentrationen aufwies:
CO-Ile-Leu 10,95 g / 100 ml und
CO-Val-Val 7,53 g / 100 ml.

Die derart hergestellten Lösungen waren in dieser Zusammensetzung bereits für die parenterale Verabreichung als Supplement für parenterale Ernährungsregime geeignet. Zur Konfektionierung wurden sie daher direkt in gespülte Glasflaschen abgefüllt, wobei vor dem Verschluß mit Gummistopfen und der Verbördelung der Glasflaschen noch der Kopfraum befüllten Glasflaschen evakuiert wurde. Die verschlossenen und verbördelten Glasflaschen wurden abschließend in einem Autoklaven 8 Minuten bei 121 °C sterilisiert.

## Patentansprüche (Patentansprüche für folgende(n) Vertragsstaat(en): AT, BE, CH, DE, DK, FR, GB, GR, IT, LI, LU, NL, SE)

1. Verbindungen der allgemeinen Formel I 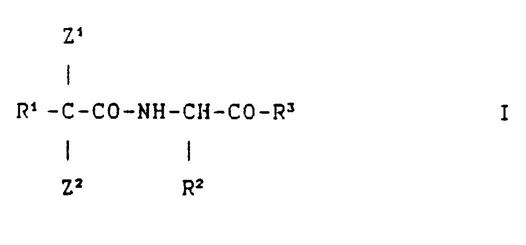 worin
R¹ für einen organischen
Rest A 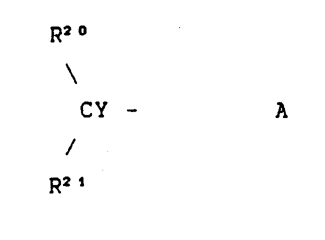 steht, worin
Y für Wasserstoff oder eine weitere Bindung steht, und falls
R²⁰ Wasserstoff ist, R²¹ Isopropyl bedeutet, und falls
R²⁰ Methyl ist, R²¹ Methyl oder Ethyl bedeutet,
R² den organischen Rest A′ 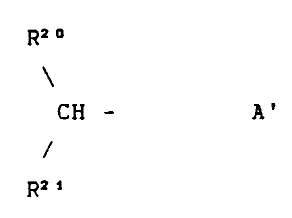 darstellt, worin R²⁰ und R²¹ obige Bedeutung besitzen,
R³ für Hydroxy oder niederes Alkoxy oder für eine Aminogruppe B 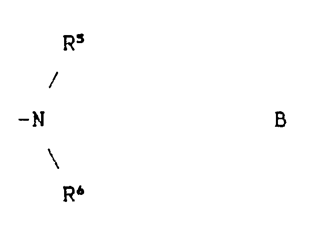 steht, worin
R⁵ Wasserstoff oder niederes Alkyl bedeutet,
R⁶ Wasserstoff, niederes Alkyl oder, falls R⁵ Wasserstoff ist, auch den Desaminorest einer biogenen Aminosäure bedeutet, oder worin
R⁵ und R⁶ gemeinsam mit dem N-Atom, an welches sie gebunden sind, einen 3- bis 6-gliedrigen Heterocyclus bilden, und
Z¹ und Z² gemeinsam für Sauerstoff oder für eine physiologisch verträgliche Alkylendioxygruppe O-(CH₂)ₙ-O stehen, worin n 1 - 4 ist, oder
Z¹ und Z² je für eine physiologisch verträgliche R⁷-O-Gruppe stehen, worin R⁷ niederes Alkyl bedeutet, oder
Z¹ für eine R⁷-O-Gruppe steht, worin R⁷ obige Bedeutung besitzt, und
Z² gemeinsam mit Y eine Bindung darstellt,
sowie Salze von solchen Verbindungen der Formel I, worin R³ für Hydroxy steht, mit einem physiologisch verträglichen Kation.

2. Verbindungen gemäß Anspruch 1, worin
Z¹ und Z² gemeinsam für Sauerstoff stehen,
R² und R³ die obige Bedeutung besitzen und
R¹ für einen organischen Rest A′ steht, worin
R²⁰ und R²¹ die obige Bedeutung besitzen.

3. Verbindungen gemäß Anspruch 1, worin
Z¹ und Z² gemeinsam für Sauerstoff oder für eine physiologisch verträgliche Alkylendioxygruppe O-(CH₂)ₙ-O stehen, worin n 1 - 4 ist, oder
Z¹ und Z² je für eine physiologisch verträgliche R⁷-O-Gruppe stehen, worin R⁷ niederes Alkyl bedeutet, vorzugsweise aber für die Ethoxygruppe stehen,
R² und R³ die obige Bedeutung, besitzen und
R¹ für einen organischen Rest A′ steht, worin
R²⁰ und R²¹ die obige Bedeutung besitzen.

4. Verbindungen gemäß Anspruch 1, worin
R² ,R³ , Z¹ und Z² die obige Bedeutung besitzen und
R¹ für einen organischen Rest A steht, worin
Y die obige Bedeutung besitzt, und worin
R²⁰ Wasserstoff und R²¹ Isopropyl bedeuten, oder worin
R²⁰ und R²¹ Methyl bedeuten.

5. Verbindungen gemäß Anspruch 1, worin
R¹ , R³ , Z¹ und Z² die obige Bedeutung besitzen und
R² für einen organischen Rest A′ steht, worin
R²⁰ Wasserstoff und R²¹ Isopropyl bedeuten, oder worin
R²⁰ und R²¹ Methyl bedeuten.

6. Verbindungen gemäß Anspruch 2, worin
R¹ und R² unabhängig voneinander für einen organischen Rest A′ stehen, worin
R²⁰ Wasserstoff und R¹ Isopropyl bedeuten, oder
R²⁰ und R²¹ Methyl bedeuten.

7. Verbindungen gemäß Anspruch 1, worin
R¹ , R² , Z¹ und Z² die obige Bedeutung besitzen und
R³ für Hydroxy steht und Salze solcher Verbindungen mit einem physiologisch verträglichen Kation.

8. Verbindungen gemäß Anspruch 7, worin das Kation ein physiologisch verträgliches Metallkation oder eine physiologisch verträgliche Ammoniumgruppe C 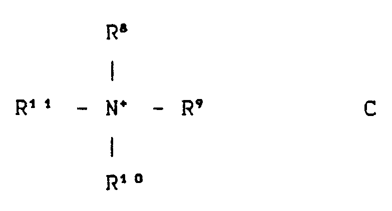 ist, worin R⁸ bis R¹¹ unabhängig voneinander Wasserstoff oder niederes Alkyl bedeuten, oder zwei der Substituenten R⁸ bis R¹¹ gemeinsam einer C₄- oder C₅-Alkylenkette und die anderen Substituenten Wasserstoff bedeuten, oder einer der Substituenten R⁸ bis R¹¹ den Desaminorest einer basischen biogenen α-Aminocarbonsäure darstellt und die anderen Substituenten Wasserstoff bedeuten.

9. Verbindungen gemäß Anspruch 8, worin das Kation ein physiologisch verträgliches Metallkation aus der Gruppe Natrium, Kalium, Calcium, Magnesium oder Zink ist.

10. Verbindungen gemäß Anspruch 8, worin das Kation für das Ammoniumion NH₄⁺ oder
für eine Ammoniumiongruppe C, in welcher R⁸ bis R¹⁰ Wasserstoff und R¹¹ den Desaminorest einer biogenen basischen α-Aminocarbonsäure aus der Gruppe Lys, Arg, His oder Orn bedeuten, steht.

11. Verwendung von Verbindungen gemäß Anspruch 1 als Pharmazeutika oder Diätetika in größeren Säugetieren, insbesondere Menschen.

12. Arzneimittel und Diätetika, welche als wirksamen Bestandteil Verbindungen gemäß Anspruch 1 neben üblichen physiologisch verträglichen Hilfs- und/oder Trägerstoffen enthalten.

13. Verfahren zur Herstellung von Verbindungen der allgemeinen Formel I 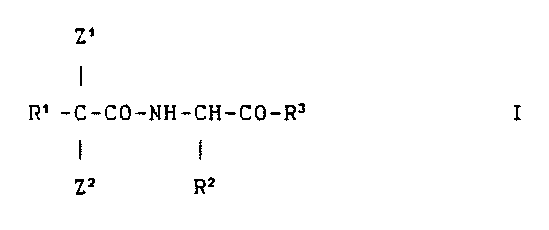 worin
R¹ für einen organischen
Rest A 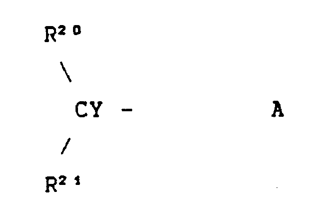 steht, worin
Y für Wasserstoff oder eine weitere Bindung steht, und falls
R²⁰ Wasserstoff ist, R²¹ Isopropyl bedeutet, und falls
R²⁰ Methyl ist, R²¹ Methyl oder Ethyl bedeutet,
R² den organischen Rest A′ 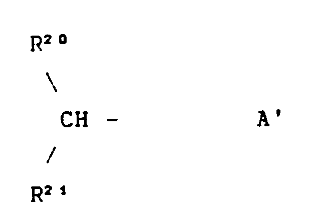 darstellt, worin R²⁰ und R²¹ obige Bedeutung besitzen,
R³ für Hydroxy oder niederes Alkoxy oder für eine Aminogruppe B 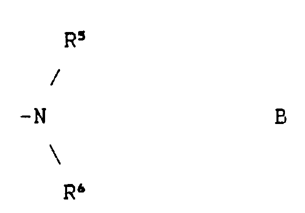 steht, worin
R⁵ Wasserstoff oder niederes Alkyl bedeutet,
R⁶ Wasserstoff, niederes Alkyl oder, falls R⁵ Wasserstoff ist, auch den Desaminorest einer biogenen Aminosäure bedeutet, oder worin
R⁵ und R⁶ gemeinsam mit dem N-Atom, an welches sie gebunden sind, einen 3- bis 6-gliedrigen Heterocyclus bilden, und
Z¹ und Z² gemeinsam für Sauerstoff oder für eine physiologisch verträgliche Alkylendioxygruppe O-(CH₂)ₙ-O stehen, worin n 1 - 4 ist, oder
Z¹ und Z² je für eine physiologisch verträgliche R⁷-O-Gruppe stehen, worin R⁷ niederes Alkyl bedeutet, oder
Z¹ für eine R⁷-O-Gruppe steht, worin R⁷ obige Bedeutung besitzt, und
Z² gemeinsam mit Y eine Bindung darstellt,
sowie Salze von solchen Verbindungen der Formel I, worin R³ für Hydroxy, steht mit einem physiologisch verträglichen Kation,
dadurch gekennzeichnet, daß man
a) zur Herstellung von Verbindungen der allgemeinen Formel Ia 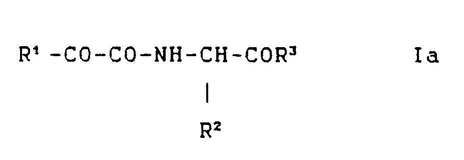 worin R¹ , R² und R³ obige Bedeutung besitzen,
in Verbindungen der allgemeinen Formel II 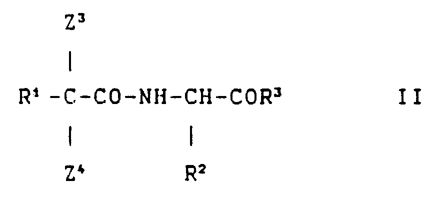 worin R¹ , R² und R³ obige Bedeutung besitzen, und Z³ und Z⁴ Alkoxy oder Alkylthio bedeuten oder gemeinsam für Alkylendioxy oder Alkylendithio stehen, oder worin
Z³ Alkoxy oder Alkylthio bedeutet und Z⁴ gemeinsam mit Y eine Bindung darstellt,
die CZ³Z⁴-Gruppierung in eine Ketogruppe überführt oder
b) zur Herstellung von Säuren der allgemeinen Formel Ib 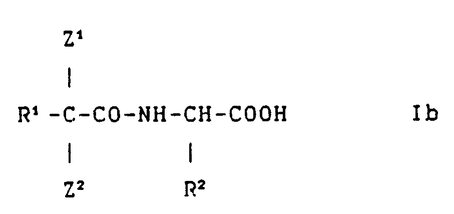 worin R¹ , R² , Z¹ und Z² obige Bedeutung besitzen,
Ester der allgemeinen Formel Ic 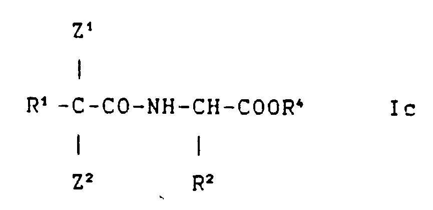 worin R¹ , R² , Z¹ und Z² obige Bedeutung besitzen und R⁴ niederes Alkyl bedeutet,
hydrolysiert oder
c) zur Herstellung von Verbindungen der allgemeinen Formel Id 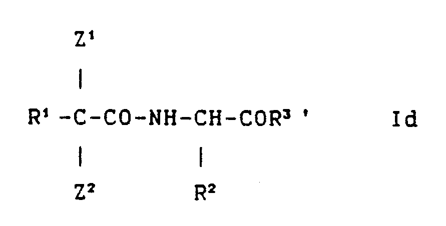 worin Z¹ , Z² , R¹ und R² obige Bedeutung besitzen und R³′ niederes Alkoxy oder eine R⁵′ R⁶′-N-Gruppe darstellt, worin R⁵′ und R⁶′ die für R⁵ und R⁶ angegebene Bedeutung mit Ausnahme von Wasserstoff besitzen,
Verbindungen der allgemeinen Formel III 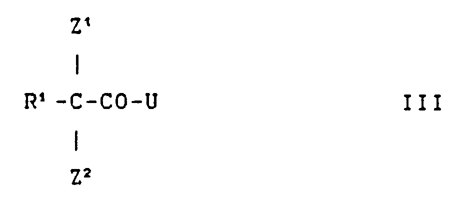 worin R¹ , Z¹ und Z² obige Bedeutung besitzen, und U ein reaktiver Rest ist,
mit Verbindungen der allgemeinen Formel IV 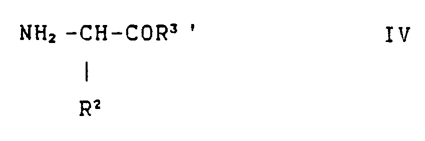 worin R² und R³′ obige Bedeutung besitzen,
umsetzt oder
d) zur Herstellung von Verbindungen der allgemeinen Formel Ie 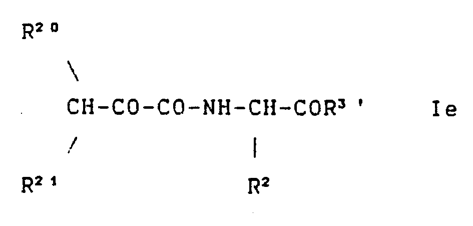 worin R²⁰ , R²¹ , R² und R³′ obige Bedeutung besitzen,
Verbindungen der allgemeinen Formel V 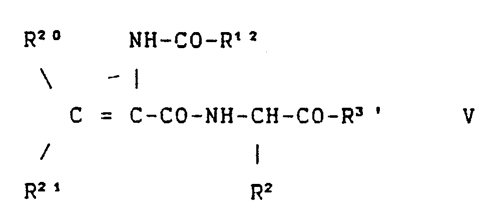 worin R²⁰ , R²¹ , R² und R³′ obige Bedeutung besitzen und R¹² niederes Alkyl, Phenyl, Trifluormethyl oder Trichlormethyl bedeutet,
spaltet oder
e) Säuren der allgemeinen Formel Ib oder deren reaktionsfähige Säurederivate mit Aminen der allgemeinen Formel VI 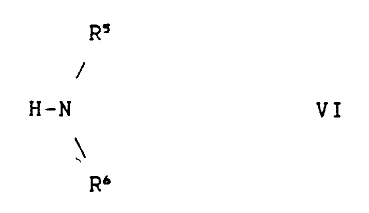 worin R⁵ und R⁶ obige Bedeutung besitzen,
umsetzt zu Verbindungen der allgemeinen Formel If 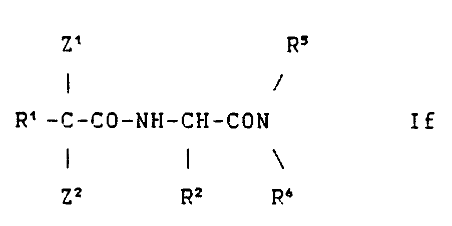 worin R¹ , R² , R⁵ , R⁶ , Z¹ und Z² obige Bedeutung besitzen,
und gewünschtenfalls Säuren der Formel Ib in ihre Salze mit physiologisch verträglichen Kationen überführt oder Salze der Säuren der Formel Ib in die freien Säuren überführt.

## Patentansprüche (Patentansprüche für folgende(n) Vertragsstaat(en): ES)

1. Verfahren zur Herstellung von Verbindungen der allgemeinen Formel I 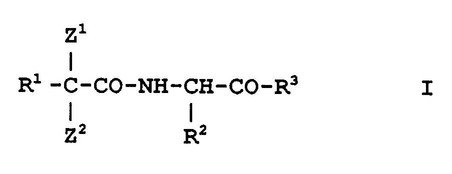 worin
R¹ für einen organischen
Rest A 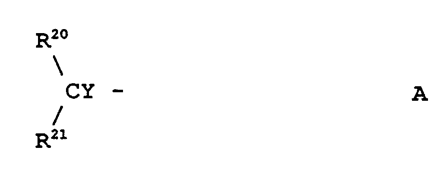 steht, worin
Y für Wasserstoff oder eine weitere Bindung steht, und falls
R²⁰ Wasserstoff ist, R²¹ Isopropyl bedeutet, und falls
R²⁰ Methyl ist, R²¹ Methyl oder Ethyl bedeutet,
R² den organischen Rest A' 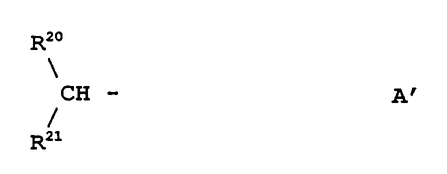 darstellt, worin R²⁰ und R²¹ obige Bedeutung besitzen,
R³ für Hydroxy oder niederes Alkoxy oder für eine Aminogruppe B 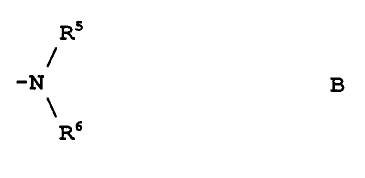 steht, worin
R⁵ Wasserstoff oder niederes Alkyl bedeutet,
R⁶ Wasserstoff, niederes Alkyl oder, falls R⁵ Wasserstoff ist, auch den Desaminorest einer biogenen Aminosäure bedeutet, oder worin
R⁵ und R⁶ gemeinsam mit dem N-Atom, an welches sie gebunden sind, einen 3- bis 6-gliedrigen Heterocyclus bilden, und
Z¹ und Z² gemeinsam für Sauerstoff oder für eine physiologisch verträgliche Alkylendioxygruppe O-(CH₂)ₙ-O stehen, worin n 1 - 4 ist, oder
Z¹ und Z² je für eine physiologisch verträgliche R⁷-O-Gruppe stehen, worin R⁷ niederers Alkyl bedeutet, oder
Z¹ für eine R⁷-O-Gruppe steht, worin R⁷ obige Bedeutung besitzt, und
Z² gemeinsam mit Y eine Bindung darstellt,
sowie Salze von solchen Verbindungen der Formel I, worin R³ für Hydroxy steht, mit einem physiologisch verträglichen Kation,
dadurch gekennzeichnet, daß man
a) zur Herstellung von Verbindungen der allgemeinen Formel Ia 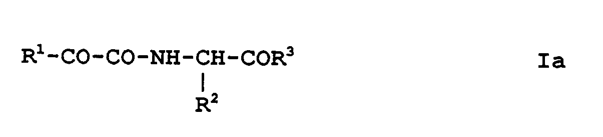 worin R¹, R² und R³ obige Bedeutung besitzen,
in Verbindung der allgemeinen Formel II 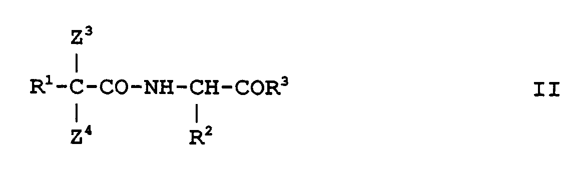 worin R¹, R² und R³ obige Bedeutung besitzen, und Z³ und Z⁴ Alkoxy oder Alkylthio bedeuten oder gemeinsam für Alkylendioxy oder Alkylendithio stehen, oder worin
Z³ Alkoxy oder Alkylthio bedeutet und Z⁴ gemeinsam mit Y eine Bindung darstellt,
die CZ³Z⁴-Gruppierung in eine Ketogruppe überführt oder
b) zur Herstellung von Säuren der allgemeinen Formel Ib 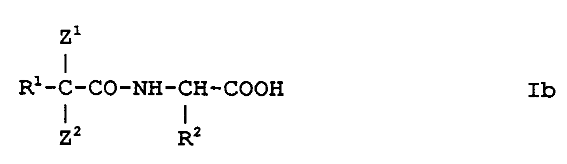 worin R¹, R², Z¹ und Z² obige Bedeutung besitzen,
Ester der allgemeinen Formel Ic 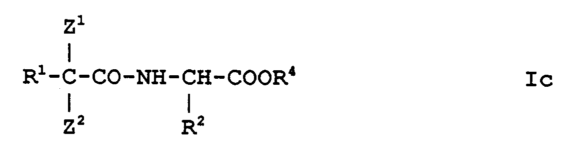 worin R¹, R², Z¹ und Z² obige Bedeutung besitzen und R⁴ niederes Alkyl bedeutet,
hydrolysiert oder
c) zur Herstellung von Verbindungen der allgemeinen Formel Id 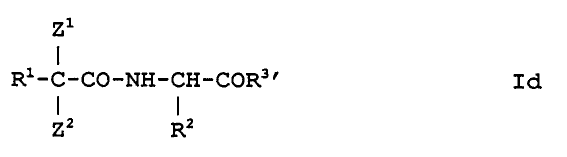 worin Z¹, Z², R¹ und R² obige Bedeutung besitzen und R³' niederes Alkoxy oder eine R⁵'R⁶'-N-Gruppe darstellt, worin R⁵' und R⁶' die für R⁵ und R⁶ angegebene Bedeutung mit Ausnahme von Wasserstoff besitzen,
Verbindungen der allgemeinen Formel III 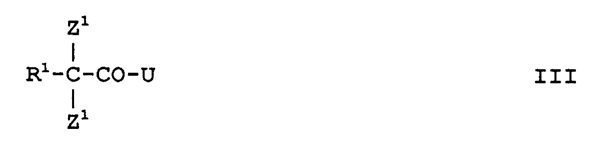 worin R¹, Z¹ und Z² obige Bedeutung besitzen, und U ein reaktiver Rest ist,
mit Verbindungen der allgemeinen Formel IV 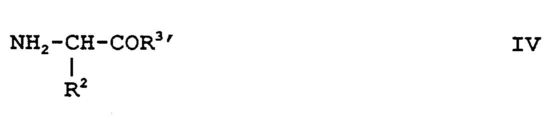 worin R² und R³' obige Bedeutung besitzen,
umsetzt oder
d) zur Herstellung von Verbindungen der allgemeinen Formel Ie 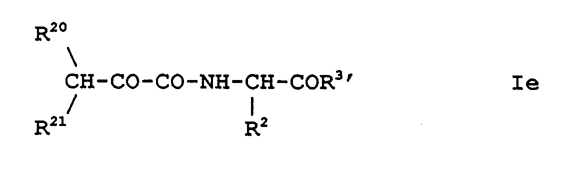 worin R²⁰, R²¹, R² und R³' obige Bedeutung besitzen,
Verbindungen der allgemeinen Formel V 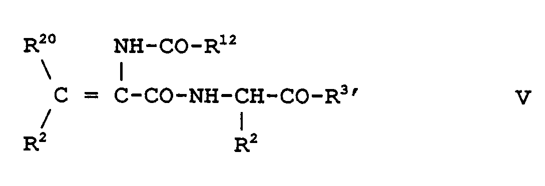 worin R²⁰, R²¹, R² und R³' obige Bedeutung besitzen und R¹² niederes Alkyl, Phenyl, Trifluormethyl oder Trichlormethyl bedeutet,
spaltet oder
e) Säuren der allgemeinen Formel Ib oder deren reaktionsfähige Säurederivate mit Aminen der allgemeinen Formel VI 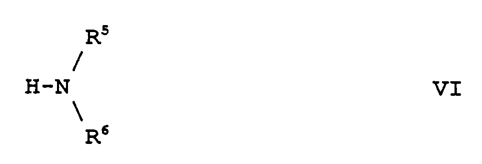 worin R⁵ und R⁶ obige Bedeutung besitzen,
umsetzt zu Verbindungen der allgemeinen Formel If 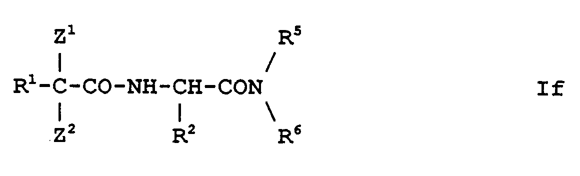 worin R¹, R², R⁵, R⁶, Z¹ und Z² obige Bedeutung besitzen,
und gewünschtenfalls Säuren der Formel Ib in ihre Salze mit physiologisch verträglichen Kationen überführt oder Salze der Säuren der Formel Ib in die freien Säuren überführt.

2. Verfahren gemäß Anspruch 1 zur Herstellung von Verbindungen der Formel I, worin
Z¹ und Z² gemeinsam für Sauerstoff stehen,
R² und R³ die obige Bedeutung besitzen und
R¹ für einen organischen Rest A' steht, worin
R²⁰ und R²¹ die obige Bedeutung besitzen.

3. Verfahren gemäß Anspruch 1 zur Herstellung von Verbindungen der Formel I, worin
Z¹ und Z² gemeinsam für Sauerstoff oder für eine physiologisch verträgliche Alkylendioxygruppe O-(CH₂)ₙ-O stehen, worin n 1 - 4 ist, oder
Z¹ und Z² je für eine physiologisch verträgliche R⁷-O-Gruppe stehen, worin R⁷ niederes Alkyl bedeutet, vorzugsweise aber für die Ethoxygruppe stehen,
R² und R³ die obige Bedeutung besitzen und
R¹ für einen organischen Rest A' steht, worin
R²⁰ und R²¹ die obige Bedeutung besitzen.

4. Verfahren gemäß Anspruch 1 zur Herstellung von Verbindungen der Formel I, worin
R², R³, Z¹ und Z² die obige Bedeutung besitzen und
R¹ für einen organischen Rest A steht, worin
Y die obige Bedeutung besitzt, und worin
R²⁰ Wasserstoff und R²¹ Isopropyl bedeuten, oder worin
R²⁰ und R²¹ Methyl bedeuten.

5. Verfahren gemäß Anspruch 1 zur Herstellung von Verbindungen der Formel I, worin
R¹, R³, Z¹ und Z² die obige Bedeutung besitzen und
R² für einen organischen Rest A' steht, worin
R²⁰ Wasserstoff und R²¹ Isopropyl bedeuten, oder worin
R²⁰ und R²¹ Methyl bedeuten.

6. Verfahren gemäß Anspruch 2 zur Herstellung von Verbindudngen der Formel I, worin
R¹ und R² unabhängig voneinander für einen organischen Rest A' stehen, worin
R²⁰ Wasserstoff und R¹ Isopropyl bedeuten, oder
R²⁰ und R²¹ Methyl bedeuten.

7. Verfahren gemäß Anspruch 1 zur Herstellung von Verbindungen der Formel I, worin
R¹, R², Z¹ und Z² die obige Bedeutung besitzen und
R³ für Hydroxy steht und Salze solcher Verbindungen mit einem physiologisch verträglichen Kation.

8. Verfahren gemäß Anspruch 7 zur Herstellung von Verbindungen der Formel I, worin das Kation ein physiologisch verträgliches Metallkation oder eine physiologisch verträgliche Ammoniumgruppe C 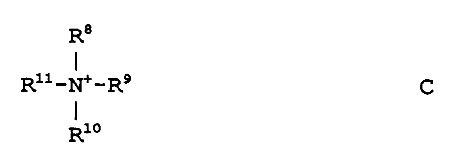 ist, worin R⁸ bis R¹¹ unabhängig voneinander Wasserstoff oder niederes Alkyl bedeuten, oder zwei der Substituenten R⁸ bis R¹¹ gemeinsam eine C₄- oder C₅-Alkylenkette und die anderen Substituenten Wasserstoff bedeuten, oder einer der Substituenten R⁸ bis R¹¹ den Desaminorest einer basischen biogenen α-Aminocarbonsäure darstellt und die anderen Substituenten Wasserstoff bedeuten.

9. Verfahren gemäß Anspruch 8 zur Herstellung von Verbindungen der Formel I, worin das Kation ein physiologisch verträgliches Metallkation aus der Gruppe Natrium, Kalium, Calcium, Magnesium oder Zink ist.

10. Verfahren gemäß Anspruch 8 zur Herstellung von Verbindungen der Formel I, worin das Kation für
das Ammoniumion NH₄⁺ oder
für eine Ammoniumiongruppe C, in welcher R⁸ bis R¹⁰ Wasserstoff und R¹¹ den Desaminorest einer biogenen basischen α-Aminocarbonsäure aus der Gruppe Lys, Arg, His oder Orn bedeuten, steht.

## Claims (Claims for the following Contracting State(s): AT, BE, CH, DE, DK, FR, GB, GR, IT, LI, LU, NL, SE)

1. Compounds of the general formula I 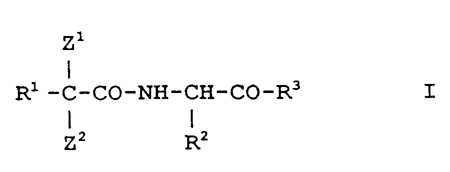 in which
R¹ stands for an organic radical A 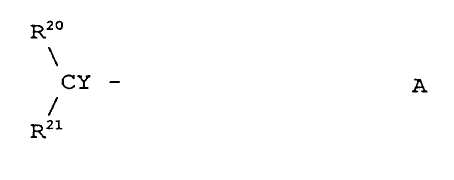 in which
Y stands for hydrogen or another bond, and, if
R²⁰ is hydrogen, R²¹ denotes isopropyl, and, if
R²⁰ is methyl, R²¹ denotes methyl or ethyl,
R² represents the organic radical A' 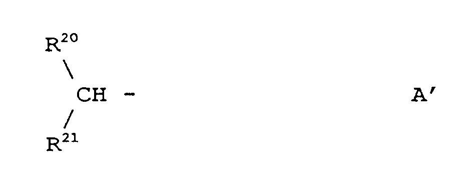 in which R²⁰ and R²¹ have the above meanings,
R³ stands for hydroxy or lower alkoxy or for an amino group B 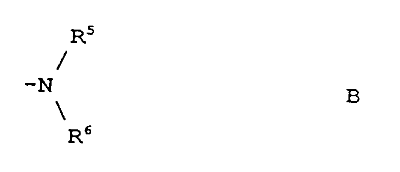 in which
R⁵ denotes hydrogen or lower alkyl,
R⁶ denotes hydrogen, lower alkyl or, if R⁵ is hydrogen, also the deamino radical of a biogenic amino acid, or in which
R⁵ and R⁶, together with the N atom to which they are bonded, form a 3- to 6-membered heterocycle, and
Z¹ and Z² together stand for oxygen or for a physiologically compatible alkylenedioxy group O-(CH₂)ₙ-O in which n is 1 - 4, or
Z¹ and Z² each stand for a physiologically compatible R⁷-O- group in which R⁷ denotes lower alkyl, or
Z¹ stands for an R⁷-O- group in which R⁷ has the above meaning, and
Z² together with Y represents a bond,
and salts of those compounds of formula I in which R³ stands for hydroxy with a physiologically compatible cation.

2. Compounds according to Claim 1, in which
Z¹ and Z² together stand for oxygen,
R² and R³ have the above meanings, and
R¹ stands for an organic radical A' in which
R²⁰ and R²¹ have the above meanings.

3. Compounds according to Claim 1, in which
Z¹ and Z² together stand for oxygen or for a physiologically compatible alkylenedioxy group O-(CH₂)ₙ-O in which n is 1 - 4, or
Z¹ and Z² each stand for a physiologically compatible R⁷-O- group in which R⁷ denotes lower alkyl, but preferably stand for the ethoxy group,
R² and R³ have the above meanings, and
R¹ stands for an organic radical A' in which
R²⁰ and R²¹ have the above meanings.

4. Compounds according to Claim 1, in which
R², R³, Z¹ and Z² have the above meanings, and
R¹ stands for an organic radical A in which
Y has the above meaning, and in which
R²⁰ denotes hydrogen and R²¹ denotes isopropyl, or in which
R²⁰ and R²¹ denote methyl.

5. Compounds according to Claim 1, in which
R¹, R³, Z¹ and Z² have the above meanings, and
R² stands for an organic radical A' in which
R²⁰ denotes hydrogen and R²¹ denotes isopropyl, or in which
R²⁰ and R²¹ denote methyl.

6. Compounds according to Claim 2, in which
R¹ and R² stand, independently of one another, for an organic radical A' in which
R²⁰ denotes hydrogen and R¹ denotes isopropyl, or
R²⁰ and R²¹ denote methyl.

7. Compounds according to Claim 1, in which
R¹, R², Z¹ and Z² have the above meanings, and
R³ stands for hydroxy, and salts of such compounds with a physiologically compatible cation.

8. Compounds according to Claim 7, in which the cation is a physiologically compatible metal cation or a physiologically compatible ammonium group C 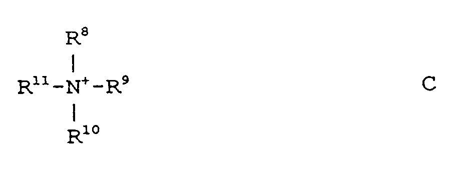 in which R⁸ to R¹¹ denote, independently of one another, hydrogen or lower alkyl, or two of the substituents R⁸ to R¹¹ together denote a C₄- or C₅- alkylene chain and the other substituents denote hydrogen, or one of the substituents R⁸ to R¹¹ represents the deamino radical of a basic biogenic α-amino carboxylic acid, and the other substituents denote hydrogen.

9. Compounds according to Claim 8, in which the cation is a physiologically compatible metal cation from the group comprising sodium, potassium, calcium, magnesium or zinc.

10. Compounds according to Claim 8, in which the cation stands for the ammonium ion NH₄⁺ or for an ammonium ion group C in which R⁸ to R¹⁰ denote hydrogen and R¹¹ denotes the deamino radical of a biogenic basic α-amino carboxylic acid from the group comprising Lys, Arg, His or Orn.

11. The use of compounds according to Claim 1 as pharmaceuticals or dietetics in larger mammals, especially humans.

12. Pharmaceuticals and dietetics which contain as active constituent compounds according to Claim 1 in addition to customary physiologically compatible auxiliaries and/or vehicles.

13. A process for the preparation of compounds of the general formula I 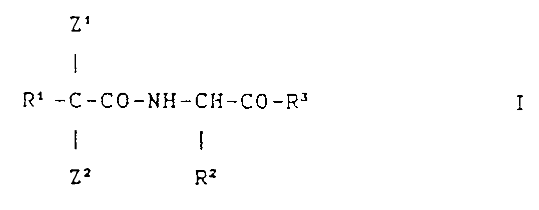 in which
R¹ stands for an organic radical A 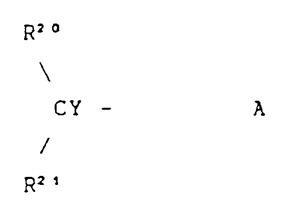 in which
Y stands for hydrogen or another bond and, if
R²⁰ is hydrogen, R²¹ denotes isopropyl and, if
R²⁰ is methyl, R²¹ denotes methyl or ethyl,
R² represents the organic radical A' 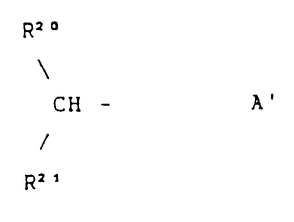 in which
R²⁰ and R²¹ have the above meanings,
R³ stands for hydroxy or lower alkoxy or for an amino group B 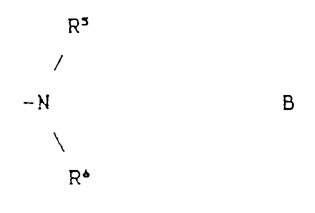 in which
R⁵ denotes hydrogen or lower alkyl,
R⁶ denotes hydrogen, lower alkyl or, if R⁵ is hydrogen, also the deamino radical of a biogenic amino acid, or in which
R⁵ and R⁶, together with the N atom to which they are bonded, form a 3- to 6-membered heterocycle, and
Z¹ and Z² together stand for oxygen or for a physiologically compatible alkylenedioxy group O-(CH₂)ₙ-O in which n is 1 - 4, or
Z¹ and Z² each stand for a physiologically compatible R⁷-O- group in which R⁷ denotes lower alkyl, or
Z¹ stands for an R⁷-O- group in which R⁷ has the above meaning, and
Z² together with Y represents a bond,
and salts of those compounds of formula I in which R³ stands for hydroxy with a physiologically compatible cation,
characterised in that
a) for the preparation of compounds of the general formula Ia 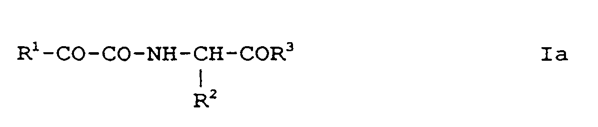 in which R¹, R² and R³ have the above meanings,
the CZ³Z⁴ group in compounds of the general formula II 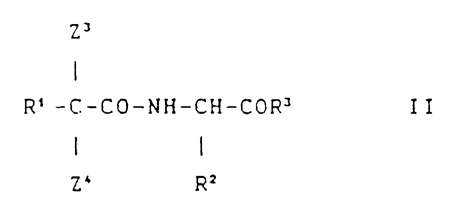 in which R¹, R² and R³ have the above meanings, and Z³ and Z⁴ denote alkoxy or alkylthio or together stand for alkylenedioxy or alkylenedithio, or in which
Z³ denotes alkoxy or alkylthio, and Z⁴ together with Y represents a bond,
is converted into a keto group or
b) for the preparation of acids of the general formula Ib 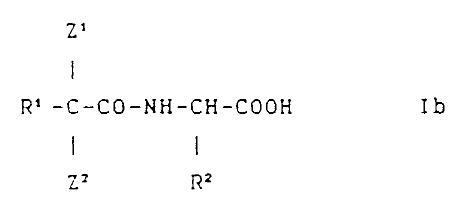 in which R¹, R², Z¹ and Z² have the above meanings,
esters of the general formula Ic 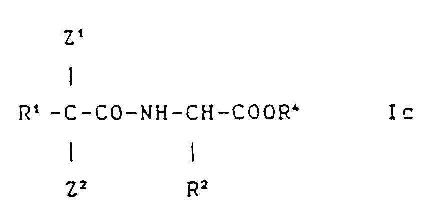 in which R¹, R², Z¹ and Z² have the above meanings, and R⁴ denotes lower alkyl,
are hydrolysed or
c) for the preparation of compounds of the general formula Id 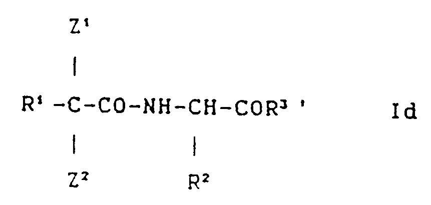 in which Z¹, Z², R¹ and R² have the above meanings, and R^{3'} represents lower alkoxy or an R^{5'}R^{6'}-N- group in which R^{5'} and R^{6'} have the meanings indicated for R⁵ and R⁶ with the exception of hydrogen,
compounds of the general formula III 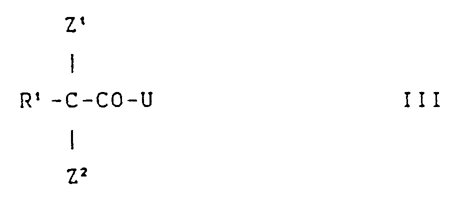 in which R¹, Z¹ and Z² have the above meanings, and U is a reactive radical,
are reacted with compounds of the general formula IV 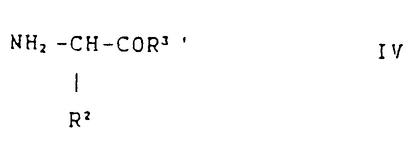 in which R² and R^{3'} have the above meanings, or
d) for the preparation of compounds of the general formula Ie 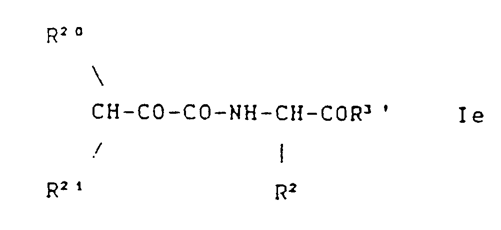 in which R²⁰, R²¹, R² and R^{3'} have the above meanings,
compounds of the general formula V 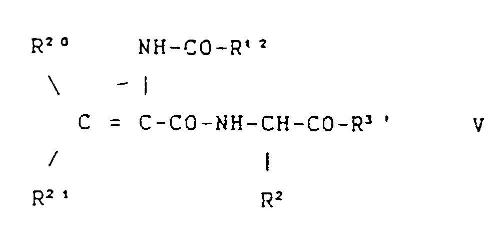 in which R²⁰, R²¹, R² and R^{3'} have the above meanings, and R¹² denotes lower alkyl, phenyl, trifluoromethyl or trichloromethyl,
are cleaved or
e) acids of the general formula Ib or their reactive acid derivatives are reacted with amines of the general formula VI 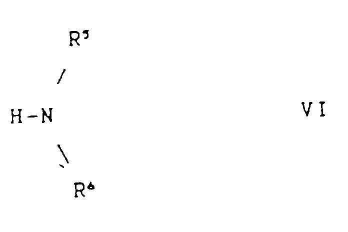 in which R⁵ and R⁶ have the above meanings,
to give compounds of the general formula If 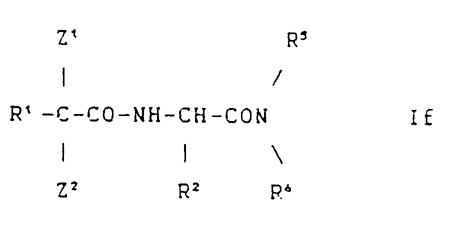 in which R¹, R², R⁵, R⁶, Z¹ and Z² have the above meanings,
and, if desired, acids of formula Ib are converted into their salts with physiologically compatible cations, or salts of the acids of formula Ib are converted into the free acids.

## Claims (Claims for the following Contracting State(s): ES)

1. A process for the preparation of compounds of the general formula I 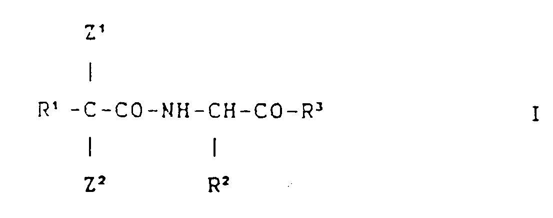 in which
R¹ stands for an organic radical A 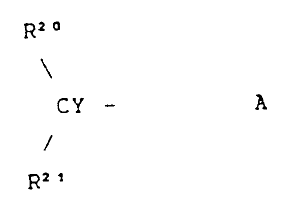 in which
Y stands for hydrogen or another bond and, if
R²⁰ is hydrogen, R²¹ denotes isopropyl and, if
R²⁰ is methyl, R²¹ denotes methyl or ethyl,
R² represents the organic radical A' 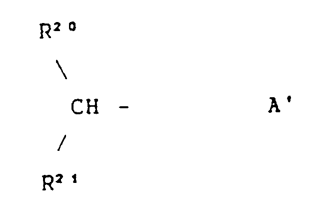 in which
R²⁰ and R²¹ have the above meanings,
R³ stands for hydroxy or lower alkoxy or for an amino group B 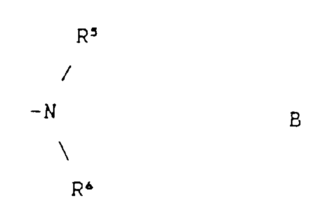 in which
R⁵ denotes hydrogen or lower alkyl,
R⁶ denotes hydrogen, lower alkyl or, if R⁵ is hydrogen, also the deamino radical of a biogenic amino acid, or in which
R⁵ and R⁶, together with the N atom to which they are bonded, form a 3- to 6-membered heterocycle, and
Z¹ and Z² together stand for oxygen or for a physiologically compatible alkylenedioxy group O-(CH₂)ₙ-O in which n is 1 - 4, or
Z¹ and Z² each stand for a physiologically compatible R⁷-O- group in which R⁷ denotes lower alkyl, or
Z¹ stands for an R⁷-O- group in which R⁷ has the above meaning, and
Z² together with Y represents a bond,
and salts of those compounds of formula I in which R³ stands for hydroxy with a physiologically compatible cation,
characterised in that
a) for the preparation of compounds of the general formula Ia 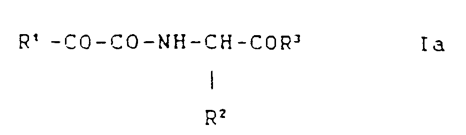 in which R¹, R² and R³ have the above meanings,
the CZ³Z⁴ group in compounds of the general formula II 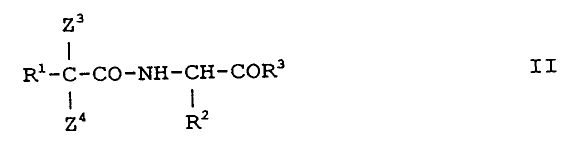 in which R¹, R² and R³ have the above meanings, and Z³ and Z⁴ denote alkoxy or alkylthio or together stand for alkylenedioxy or alkylenedithio, or in which
Z³ denotes alkoxy or alkylthio, and Z⁴ together with Y represents a bond,
is converted into a keto group or
b) for the preparation of acids of the general formula Ib 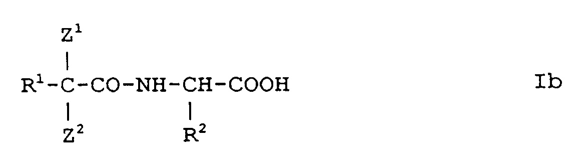 in which R¹, R², Z¹ and Z² have the above meanings,
esters of the general formula Ic 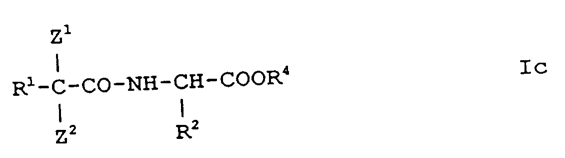 in which R¹, R², Z¹ and Z² have the above meanings, and R⁴ denotes lower alkyl,
are hydrolysed or
c) for the preparation of compounds of the general formula Id 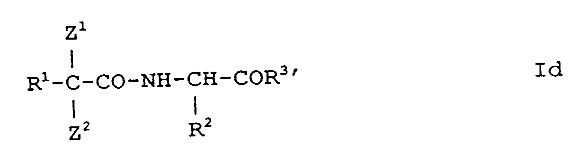 in which Z¹, Z², R¹ and R² have the above meanings, and R^{3'} represents lower alkoxy or an R^{5'}R^{6'}-N- group in which R^{5'} and R^{6'} have the meanings indicated for R⁵ and R⁶ with the exception of hydrogen,
compounds of the general formula III 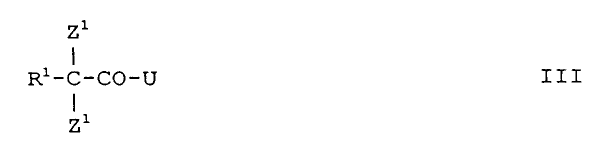 in which R¹, Z¹ and Z² have the above meanings, and U is a reactive radical,
are reacted with compounds of the general formula IV 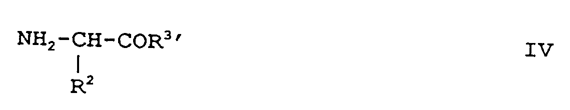 in which R² and R^{3'} have the above meanings, or
d) for the preparation of compounds of the general formula Ie 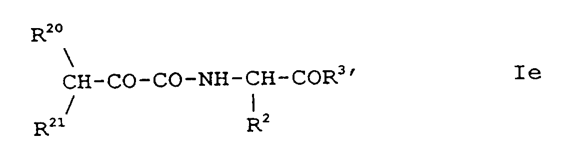 in which R²⁰, R²¹, R² and R^{3'} have the above meanings,
compounds of the general formula V 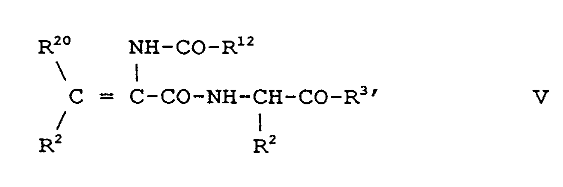 in which R²⁰, R²¹, R² and R^{3'} have the above meanings, and R¹² denotes lower alkyl, phenyl, trifluoromethyl or trichloromethyl,
are cleaved or
e) acids of the general formula Ib or their reactive acid derivatives are reacted with amines of the general formula VI 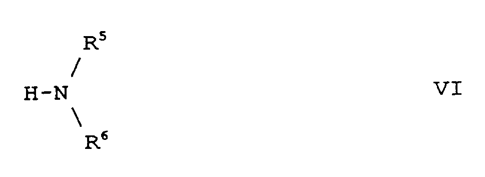 in which R⁵ and R⁶ have the above meanings,
to give compounds of the general formula If 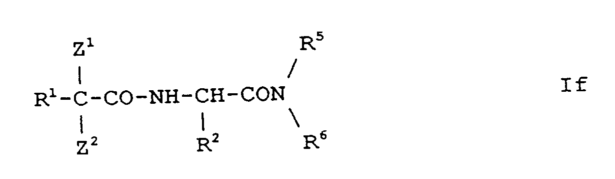 in which R¹, R², R⁵, R⁶, Z¹ and Z² have the above meanings,
and, if desired, acids of formula Ib are converted into their salts with physiologically compatible cations, or salts of the acids of formula Ib are converted into the free acids.

2. A process according to Claim 1 for the preparation of compounds of Formula I, in which
Z¹ and Z² together stand for oxygen,
R² and R³ have the above meanings, and
R¹ stands for an organic radical A' in which
R²⁰ and R²¹ have the above meanings.

3. A process according to Claim 1 for the preparation of compounds of Formula I, in which
Z¹ and Z² together stand for oxygen or for a physiologically compatible alkylenedioxy group O-(CH₂)ₙ-O in which n is 1 - 4, or
Z¹ and Z² each stand for a physiologically compatible R⁷-O- group in which R⁷ denotes lower alkyl, but preferably stand for the ethoxy group,
R² and R³ have the above meanings, and
R¹ stands for an organic radical A' in which
R²⁰ and R²¹ have the above meanings.

4. A process according to Claim 1 for the preparation of compounds of Formula I, in which
R², R³, Z¹ and Z² have the above meanings, and
R¹ stands for an organic radical A in which
Y has the above meaning, and in which
R²⁰ denotes hydrogen and R²¹ denotes isopropyl, or in which
R²⁰ and R²¹ denote methyl.

5. A process according to Claim 1 for the preparation of compounds of Formula I, in which
R¹, R³, Z¹ and Z² have the above meanings, and
R² stands for an organic radical A' in which
R²⁰ denotes hydrogen and R²¹ denotes isopropyl, or in which
R²⁰ and R²¹ denote methyl.

6. A process according to Claim 2 for the preparation of compounds of Formula I, in which
R¹ and R² stand, independently of one another, for an organic radical A' in which
R²⁰ denotes hydrogen and R¹ denotes isopropyl, or
R²⁰ and R²¹ denote methyl.

7. A process according to Claim 1 for the preparation of compounds of Formula I, in which
R¹, R², Z¹ and Z² have the above meanings, and
R³ stands for hydroxy, and salts of such compounds with a physiologically compatible cation.

8. A process according to Claim 7 for the preparation of compounds of Formula I, in which the cation is a physiologically compatible metal cation or a physiologically compatible ammonium group C 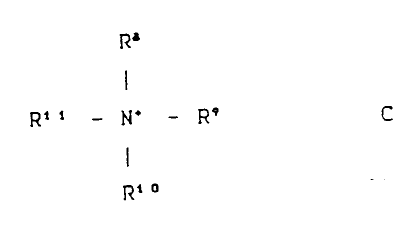 in which R⁸ to R¹¹ denote, independently of one another, hydrogen or lower alkyl, or two of the substituents R⁸ to R¹¹ together denote a C₄- or C₅- alkylene chain and the other substituents denote hydrogen, or one of the substituents R⁸ to R¹¹ represents the deamino radical of a basic biogenic α-amino carboxylic acid, and the other substituents denote hydrogen.

9. A process according to Claim 8 for the preparation of compounds of Formula I, in which the cation is a physiologically compatible metal cation from the group comprising sodium, potassium, calcium, magnesium or zinc.

10. A process according to Claim 8 for the preparation of compounds of Formula I, in which the cation stands for the ammonium ion NH₄⁺ or
for an ammonium ion group C in which R⁸ to R¹⁰ denote hydrogen and R¹¹ denotes the deamino radical of a biogenic basic α-amino carboxylic acid from the group comprising Lys, Arg, His or Orn.

## Revendications (Revendications pour l'(les) Etat(s) contractant(s) suivant(s): AT, BE, CH, DE, DK, FR, GB, GR, IT, LI, LU, NL, SE)

1. Composés de formule générale I 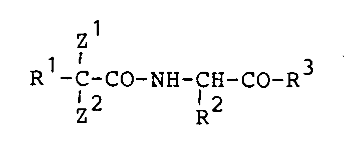 dans laquelle
R¹ représente un radical organique A 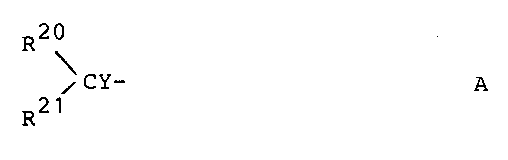 dans lequel
Y représente un atome d'hydrogène ou une autre liaison, et lorsque
R²⁰ est un atome d'hydrogène, R²¹ représente le groupe isopropyle, et lorsque
R²⁰ est le groupe méthyle, R²¹ représente le groupe méthyle ou éthyle,
R² représente le radical organique A' 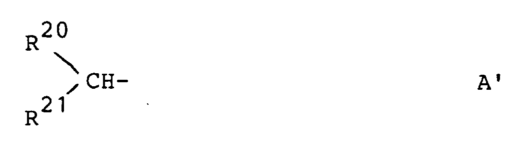 dans lequel R²⁰ et R²¹ ont les significations données ci-dessus,
R³ représente le groupe hydroxy ou un groupe alcoxy inférieur ou un groupe amino B 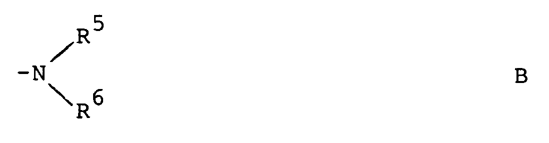 dans lequel
R⁵ représente un atome d'hydrogène ou un groupe alkyle inférieur,
R⁶ représente un atome d'hydrogène, un groupe alkyle inférieur ou, lorsque R⁵ est un atome d'hydrogène, également le reste désamino d'un aminoacide biogène,
ou dans lequel
R⁵ et R⁶ forment ensemble avec l'atome d'azote auquel ils sont liés, un hétérocycle à 3-6 chaînons, et
Z¹ et Z² représentent ensemble un atome d'oxygène ou un groupe alkylènedioxy O-(CH₂)ₙ-O physiologiquement acceptable, dans lequel n va de 1 à 4, ou
Z¹ et Z² représentent chacun un groupe R⁷-O physiologiquement acceptable, dans lequel R⁷ représente un groupe alkyle inférieur, ou
Z¹ représente un groupe R⁷-O-, dans lequel R⁷ a la signification donnée ci-dessus, et
Z² conjointement avec Y représente une liaison, et sels des composés de formule I, dans lesquels R³ représente le groupe hydroxy, avec un cation physiologiquement acceptable.

2. Composés selon la revendication 1, dans lesquels
Z¹ et Z² représentent ensemble un atome d'oxygène,
R² et R³ ont les significations données plus haut, et
R¹ représente un radical organique A' dans lequel
R²⁰ et R²¹ ont les significations données plus haut.

3. Composés selon la revendication 1, dans lesquels
Z¹ et Z² représentent ensemble un atome d'oxygène ou un groupe alkylènedioxy O-(CH₂)ₙ-O physiologiquement acceptable, dans lequel n va de 1 a 4, ou
Z¹ et Z² représentent chacun un groupe R⁷-O physiologiquement acceptable, dans lequel R⁷ représente un groupe alkyle inférieur, mais de préférence le groupe éthoxy,
R² et R³ ont les significations données plus haut, et
R¹ représente un radical organique A' dans lequel
R²⁰ et R²¹ ont les significations données plus haut.

4. Composés selon la revendication 1, dans lesquels
R², R³, Z¹ et Z² ont les significations données plus haut, et
R¹ représente un radical organique A dans lequel
Y a la signification donnée plus haut, et dans lequel
R²⁰ représente un atome d'hydrogène et R²¹ représente le groupe isopropyle, ou
R²⁰ et R²¹ représentent le groupe méthyle.

5. Composés selon la revendication 1, dans lesquels
R¹, R³, Z¹ et Z² ont les significations données plus haut, et
R² représente un radical organique A' dans lequel
R²⁰ représente un atome d'hydrogène et R²¹ représente le groupe isopropyle, ou
R²⁰ et R²¹ représentent le groupe méthyle.

6. Composés selon la revendication 2, dans lesquels
R¹ et R² représentent, indépendamment l'un de l'autre, un radical organique A' dans lequel
R²⁰ représente un atome d'hydrogène et R²¹ représente le groupe isopropyle, ou
R²⁰ et R²¹ représentent le groupe méthyle.

7. Composés selon la revendication 1, dans lesquels R¹, R², Z¹ et Z² ont les significations données plus haut, et R³ représente le groupe hydroxy,
et sels de ces composés avec un cation physiologiquement acceptable.

8. Composés selon la revendication 7, dans lesquels le cation est un cation métallique physiologiquement acceptable ou un groupe ammonium C physiologiquement acceptable, 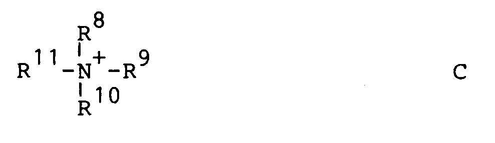 dans lequel R⁸ à R¹¹ représentent, indépendamment les uns des autres, un atome d'hydrogène ou un groupe alkyle inférieur, ou deux des substituants R⁸ à R¹¹ représentent ensemble une chaîne alkylène en C₄ ou C₅ et les autres substituants représentent des atomes d'hydrogène, ou l'un des substituants R⁸ à R¹¹ représente le reste désamino d'un acide α-aminocarboxylique basique biogène et les autres substituants représentent des atomes d'hydrogène.

9. Composés selon la revendication 8, dans lesquels le cation est un cation métallique physiologiquement acceptable choisi parmi le sodium, le potassium, le calcium, le magnésium et le zinc.

10. Composés selon la revendication 8, dans lesquels le cation représente l'ion ammonium NH₄⁺ ou un groupe ammonium C dans lequel R⁸ a R¹⁰ représentent des atomes d'hydrogène et R¹¹ représente le reste désamino d'un acide α-aminocarboxylique basique biogène choisi parmi Lys, Arg, His et Orn.

11. Utilisation des composés selon la revendication 1, en tant que produits pharmaceutiques ou diététiques chez les grands mammifères , l'homme en particulier.

12. Médicaments et produits diététiques qui contiennent, en tant que composant actif, des composés selon la revendication 1, en plus d'adjuvants et/ou de véhicules usuels, physiologiquement acceptables.

13. Procédé pour la préparation de composés de formule générale I 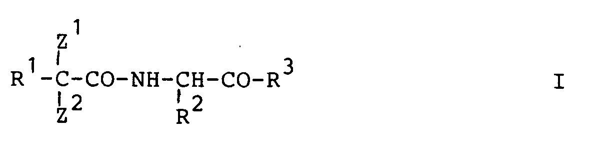 dans laquelle
R¹ représente un radical organique A 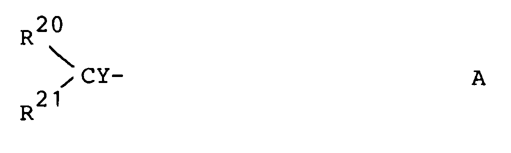 dans lequel
Y représente un atome d'hydrogène ou une autre liaison, et lorsque
R²⁰ est un atome d'hydrogène, R²¹ représente le groupe isopropyle, et lorsque
R²⁰ est le groupe méthyle, R²¹ représente le groupe méthyle ou éthyle,
R² représente le radical organique A' 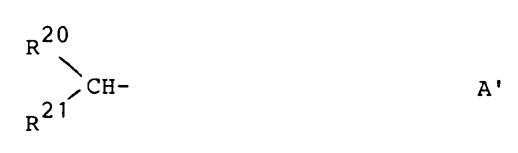 dans lequel R²⁰ et R²¹ ont les significations données ci-dessus,
R³ représente le groupe hydroxy ou un groupe alcoxy inférieur ou un groupe amino B 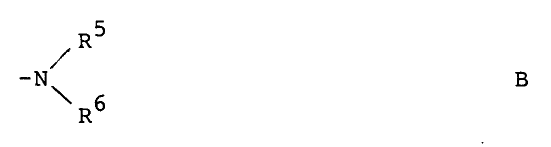 dans lequel
R⁵ représente un atome d'hydrogène ou un groupe alkyle inférieur,
R⁶ représente un atome d'hydrogène, un groupe alkyle inférieur ou, lorsque R⁵ est un atome d'hydrogène, également le reste désamino d'un aminoacide biogène,
ou dans lequel
R⁵ et R⁶ forment ensemble, avec l'atome d'azote auquel ils sont liés, un hétérocycle à 3-6 chaînons, et
Z¹ et Z² représentent ensemble un atome d'oxygène ou un groupe alkylènedioxy O-(CH₂)ₙ-O physiologiquement acceptable, dans lequel n va de 1 à 4, ou
Z¹ et Z² représentent chacun un groupe R⁷-O physiologiquement acceptable, dans lequel R⁷ représente un groupe alkyle inférieur, ou
Z¹ représente un groupe R⁷-O-, dans lequel R⁷ a la signification donnée ci-dessus, et
Z² conjointement avec Y représente une liaison,
ainsi que des sels des composés de formule I, dans lesquels R³ représente le groupe hydroxy, avec un cation physiologiquement acceptable,
caractérisé en ce que
a) pour la préparation des composés de formule générale Ia 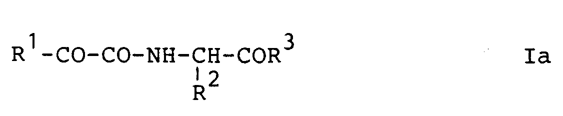 dans laquelle R¹, R² et R³ ont les significations données plus haut,
on convertit le groupement CZ³Z⁴ en un groupe céto dans des composés de formule générale II 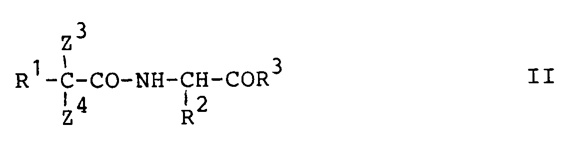 dans laquelle R¹, R² et R³ ont les significations données plus haut, et Z³ et Z⁴ représentent un groupe alcoxy ou alkylthio, ou ensemble représentent un groupe alkylènedioxy ou alkylènedithio, ou dans laquelle
Z³ représente un groupe alcoxy ou alkylthio, et Z⁴ représente, conjointement avec Y, une liaison,
ou
b) pour la préparation d'acides de formule générale Ib 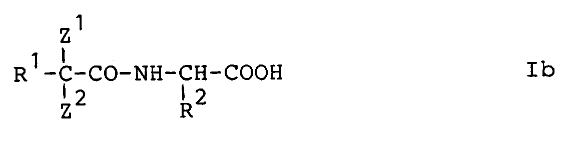 dans laquelle R¹, R², Z¹ et Z² ont les significations données plus haut,
on hydrolyse des esters de formule générale Ic 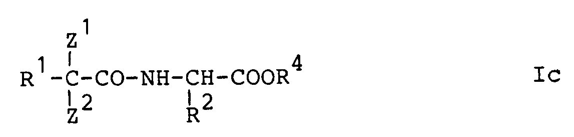 dans laquelle R¹, R², Z¹ et Z² ont les significations données plus haut, et R⁴ représente un groupe alkyle inférieur,
ou
c) pour la préparation de composés de formule générale Id 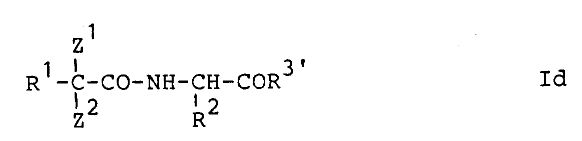 dans laquelle Z¹, Z², R¹ et R² ont les significations données plus haut, et R^{3'} représente un groupe alcoxy ou un groupe R^{5'}R^{6'}N, dans lequel R^{5'} et R^{6'} ont les significations indiquées pour R⁵ et R⁶ à l'exception de celle de l'atome d'hydrogène,
on fait réagir des composés de formule générale III 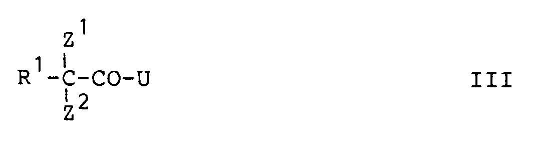 dans laquelle R¹, Z¹ et Z² ont les significations données plus haut, et U est un radical réactif,
avec des composés de formule générale IV 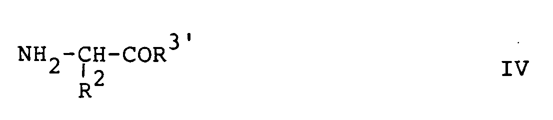 dans laquelle R² et R^{3'} ont les significations données plus haut, ou
d) pour la préparation de composés de formule générale Ie 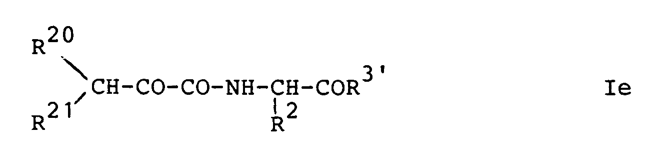 dans laquelle R²⁰, R²¹, R² et R^{3'} ont les significations données plus haut,
on scinde des composés de formule générale V 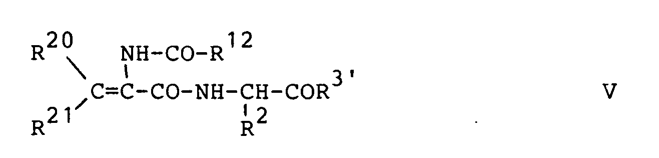 dans laquelle R²⁰, R²¹, R² et R^{3'} ont les significations données plus haut, et R¹² représente un groupe alkyle inférieur, phényle, trifluorométhyle ou trichlorométhyle,
ou
e) on fait réagir des acides de formule générale Ib, ou leurs dérivés acides réactifs, avec des amines de formule générale VI 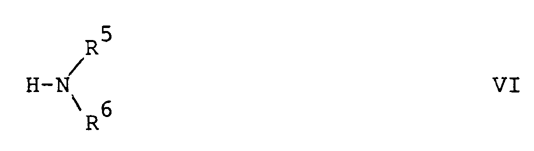 dans laquelle R⁵ et R⁶ ont les significations données plus haut,
pour aboutir à des composés de formule générale If 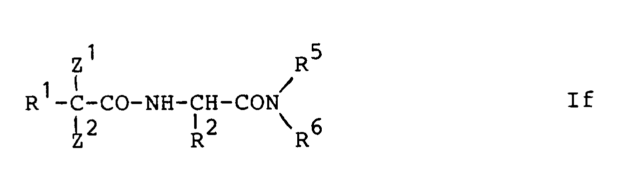 dans laquelle R¹, R², R⁵, R⁶, Z¹ et Z² ont les significations données plus haut,
et si on le désire on convertit des acides de formule Ib en leurs sels avec des cations physiologiquement acceptables, ou des sels des acides de formule Ib en acides libres.

## Revendications (Revendications pour l'(les) Etat(s) contractant(s) suivant(s): ES)

1. Procédé pour la préparation de composés de formule générale I 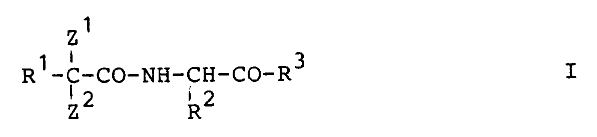 dans laquelle
R¹ représente un radical organique A 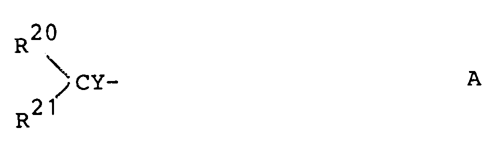 dans lequel
Y représente un atome d'hydrogène ou une autre liaison, et lorsque
R²⁰ est un atome d'hydrogène, R²¹ représente le groupe isopropyle, et lorsque
R²⁰ est le groupe méthyle, R²¹ représente le groupe méthyle ou éthyle,
R² représente le radical organique A' 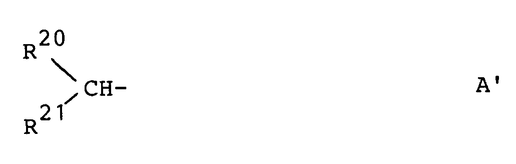 dans lequel R²⁰ et R²¹ ont les significations données ci-dessus,
R³ représente le groupe hydroxy ou un groupe alcoxy inférieur ou un groupe amino B 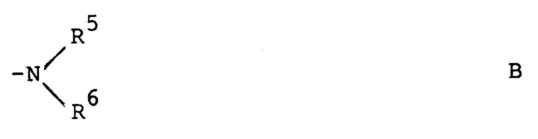 dans lequel
R⁵ représente un atome d'hydrogène ou un groupe alkyle inférieur,
R⁶ représente un atome d'hydrogène, un groupe alkyle inférieur ou, lorsque R⁵ est un atome d'hydrogène, également le reste désamino d'un aminoacide biogène,
ou dans lequel
R⁵ et R⁶ forment ensemble, avec l'atome d'azote auquel ils sont liés, un hétérocycle à 3-6 chaînons, et
Z¹ et Z² représentent ensemble un atome d'oxygène ou un groupe alkylènedioxy O-(CH₂)ₙ-O physiologiquement acceptable, dans lequel n va de 1 à 4, ou
Z¹ et Z² représentent chacun un groupe R⁷-O physiologiquement acceptable, dans lequel R⁷ représente un groupe alkyle inférieur, ou
Z¹ représente un groupe R⁷-O-, dans lequel R⁷ a la signification donnée ci-dessus, et
Z² conjointement avec Y représente une liaison,
ainsi que des sels des composés de formule I, dans lesquels R³ représente le groupe hydroxy, avec un cation physiologiquement acceptable,
caractérisé en ce que
a) pour la préparation des composés de formule générale Ia 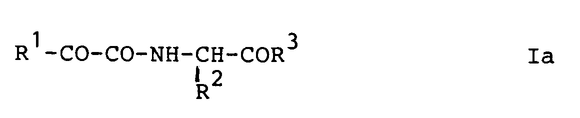 dans laquelle R¹, R² et R³ ont les significations données plus haut,
on convertit le groupement CZ³Z⁴ en un groupe céto dans des composés de formule générale II 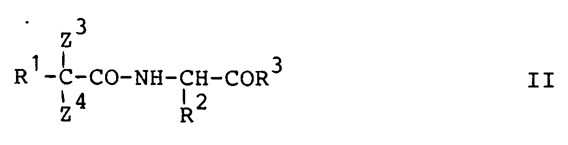 dans laquelle R¹, R² et R³ ont les significations données plus haut, et Z³ et Z⁴ représentent un groupe alcoxy ou alkylthio, ou ensemble représentent un groupe alkylènedioxy ou alkylènedithio, ou dans laquelle
Z³ représente un groupe alcoxy ou alkylthio, et Z⁴ représente, conjointement avec Y, une liaison,
ou
b) pour la préparation d'acides de formule générale Ib 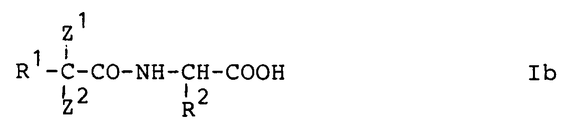 dans laquelle R¹, R², Z¹ et Z² ont les significations données plus haut,
on hydrolyse des esters de formule générale Ic 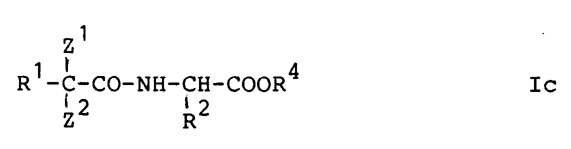 dans laquelle R¹, R², Z¹ et Z² ont les significations données plus haut, et R⁴ représente un groupe alkyle inférieur,
ou
c) pour la préparation de composés de formule générale Id 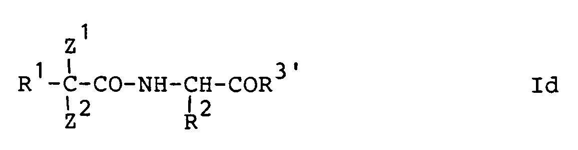 dans laquelle Z¹, Z², R¹ et R² ont les significations données plus haut, et R^{3'} représente un groupe alcoxy ou un groupe R^{5'}R^{6'}N, dans lequel R^{5'} et R^{6'} ont les significations indiquées pour R⁵ et R⁶ à l'exception de celle de l'atome d'hydrogène,
on fait réagir des composés de formule générale III 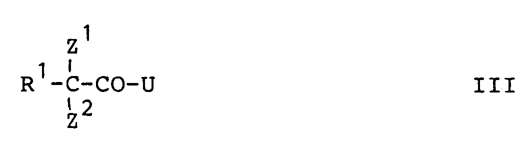 dans laquelle R¹, Z¹ et Z² ont les significations données plus haut, et U est un radical réactif,
avec des composés de formule générale IV 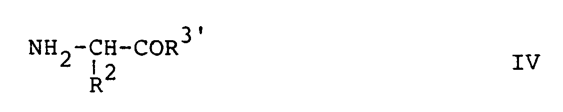 dans laquelle R² et R^{3'} ont les significations données plus haut, ou
d) pour la préparation de composés de formule générale Ie 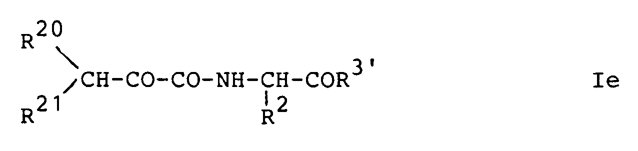 dans laquelle R²⁰, R²¹, R² et R^{3'} ont les significations données plus haut,
on scinde des composés de formule générale V 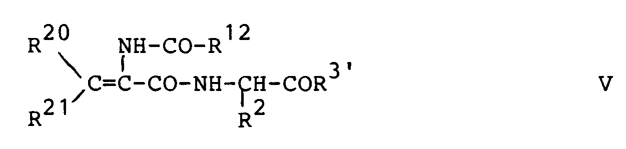 dans laquelle R²⁰, R²¹, R² et R^{3'} ont les significations données plus haut, et R¹² représente un groupe alkyle inférieur, phényle, trifluorométhyle ou trichlorométhyle,
ou
e) on fait réagir des acides de formule générale Ib, ou leurs dérivés acides réactifs, avec des amines de formule générale VI 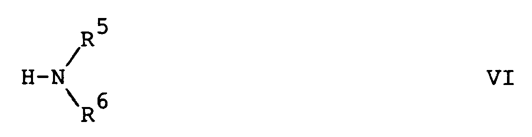 dans laquelle R⁵ et R⁶ ont les significations données plus haut,
pour aboutir à des composés de formule générale If 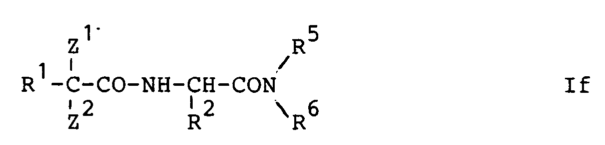 dans laquelle R¹, R², R⁵, R⁶, Z¹ et Z² ont les significations données plus haut,
et si on le désire on convertit des acides de formule Ib en leurs sels avec des cations physiologiquement acceptables, ou des sels des acides de formule Ib en les acides libres.

2. Procédé selon la revendication 1, pour la préparation de composés de formule I dans lesquels
Z¹ et Z² représentent ensemble un atome d'oxygène,
R² et R³ ont les significations données plus haut, et
R¹ représente un radical organique A' dans lequel
R²⁰ et R²¹ ont les significations données plus haut.

3. Procédé selon la revendication 1 pour la préparation de composés de formule I dans lesquels
Z¹ et Z² représentent ensemble un atome d'oxygène ou un groupe alkylènedioxy O-(CH₂)ₙ-O physiologiquement acceptable, dans lequel n va de 1 à 4, ou
Z¹ et Z² représentent chacun un groupe R⁷-O physiologiquement acceptable, dans lequel R⁷ représente un groupe alkyle inférieur, mais de préférence le groupe éthoxy,
R² et R³ ont les significations données plus haut, et
R¹ représente un radical organique A' dans lequel
R²⁰ et R²¹ ont les significations données plus haut.

4. Procédé selon la revendication 1 pour la préparation de composés de formule I dans lesquels
R², R³, Z¹ et Z² ont les significations données plus haut, et
R¹ représente un radical organique A dans lequel
Y a la signification donnée plus haut, et dans lequel
R²⁰ représente un atome d'hydrogène et R²¹ représente le groupe isopropyle, ou
R²⁰ et R²¹ représentent chacun le groupe méthyle.

5. Procédé selon la revendication 1, pour la préparation de composés de formule I dans lesquels
R¹, R³, Z¹ et Z² ont les significations données plus haut, et
R² représente un radical organique A' dans lequel
R²⁰ représente un atome d'hydrogène et R²¹ représente le groupe isopropyle, ou
R²⁰ et R²¹ représentent le groupe méthyle.

6. Procédé selon la revendication 2 pour la préparation de composés de formule I dans lesquels
R¹ et R² représentent, indépendamment l'un de l'autre, un radical organique A' dans lequel
R²⁰ représente un atome d'hydrogène et R²¹ représente le groupe isopropyle, ou
R²⁰ et R²¹ représentent le groupe méthyle.

7. Procédé selon la revendication 1, pour la préparation de composés de formule I dans lesquels
R¹, R², Z¹ et Z² ont les significations données plus haut, et
R³ représente le groupe hydroxy,
et des sels de ces composés avec un cation physiologiquement acceptable.

8. Procédé selon la revendication 7, pour la préparation de composés de formule I dans lesquels le cation est un cation métallique physiologiquement acceptable ou un groupe ammonium C physiologiquement acceptable, 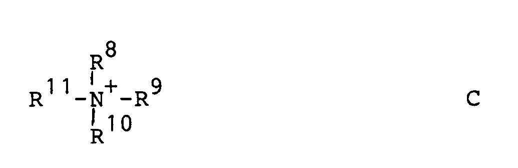 dans lequel R⁸ à R¹¹ représentent, indépendamment les uns des autres, un atome d'hydrogène ou un groupe alkyle inférieur, ou deux des substituants R⁸ à R¹¹ représentent ensemble une chaîne alkylène en C₄ ou C₅ et les autres substituants représentent des atomes d'hydrogène, ou l'un des substituants R⁸ à R¹¹ représente le reste désamino d'un acide α-aminocarboxylique basique biogène et les autres substituants représentent des atomes d'hydrogène.

9. Procédé selon la revendication 8, pour la préparation de composés de formule I dans lesquels le cation est un cation métallique physiologiquement acceptable choisi parmi le sodium, le potassium, le calcium, le magnésium et le zinc.

10. Procédé selon la revendication 1, pour la préparation de composés de formule I dans lesquels le cation représente l'ion ammonium NH₄⁺ ou un groupe ammonium C dans lequel R⁸ à R¹⁰ représentent des atomes d'hydrogène et R¹¹ représente le reste désamino d'un acide α-aminocarboxylique basique biogène choisi parmi Lys, Arg, His et Orn.
